(19) Europäisches Patentamt
European Patent Office
Office européen des brevets

(11) **EP 2 139 390 B1**

(12) **EUROPEAN PATENT SPECIFICATION**

(45) Date of publication and mention
of the grant of the patent:
**23.08.2017 Bulletin 2017/34**

(21) Application number: **06840414.4**

(22) Date of filing: **22.12.2006**

(51) Int Cl.:
**A61B 5/16** *(2006.01)*      **A61B 5/0484** *(2006.01)*

(86) International application number:
**PCT/AU2006/002006**

(87) International publication number:
**WO 2008/077178 (03.07.2008 Gazette 2008/27)**

(54) **A METHOD FOR EVALUATING THE EFFECTIVENESS OF COMMERCIAL COMMUNICATION**

VERFAHREN ZUR BEURTEILUNG DER WIRKSAMKEIT VON KOMMERZIELLER KOMMUNIKATION

PROCÉDÉ POUR ÉVALUER L'EFFICACITÉ D'UNE COMMUNICATION COMMERCIALE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**06.01.2010 Bulletin 2010/01**

(73) Proprietor: **Neuro-Insight Pty. Ltd.
Melbourne, Victoria 3122 (AU)**

(72) Inventor: **SILBERSTEIN, Richard, Bernard
Blackburn, Victoria 3122 (AU)**

(74) Representative: **Korenberg, Alexander Tal et al
Kilburn & Strode LLP
20 Red Lion Street
London WC1R 4PJ (GB)**

(56) References cited:
| | |
|---|---|
| EP-A2- 0 256 738 | WO-A1-91/09565 |
| WO-A1-99/12291 | WO-A1-99/59470 |
| US-A- 3 880 144 | US-A- 4 201 224 |
| US-A- 4 955 388 | US-A- 5 243 517 |
| US-A- 5 339 826 | US-A- 6 001 065 |
| US-A1- 2003 073 921 | US-A1- 2006 184 058 |

**Description**

BACKGROUND

The effectiveness of any piece of commercial communication or advertising depends on whether the appropriate psychological states intended by the advertiser are elicited in the viewer while they perceive the commercial communication, henceforth referred to as advertisement. For example, advertisements require a level of memory encoding at the time that the advertisers' brand appears. Frequently, the advertisement may be designed to elicit particular emotional responses in the target audience. Determining whether advertisements are effective before they are launched is most frequently carried out using a variety of psychological methodologies such as in-depth interviews and focus groups. These are widely considered inadequate in that they rely on the verbal responses of the subjects. Such verbal responses are now considered a poor indicator of emotional states and the market research industry is considering techniques that rely on brain activity measurements.

Over the last 20 years, neuroscience researchers have learnt more about the specialized role of different brain regions associated with specific psychological states or processes. By measuring the activity in different brain regions while participants perceive advertisements, it is possible to infer the psychological state elicited by the advertisement.

This disclosure relates to a method and system to identify the psychological state experienced by the subject while perceiving an advertisement. Brain activity is measured using a technique termed Steady State Probe Topography (SSPT). A method is disclosed that uses SSPT to determine the cognitive and emotional states that are elicited by the advertisements and in particular, particular points in time during the advertisement. Effectiveness is then defined in terms of whether the psychological states elicited by the advertisements are those intended by the creator of the advertisement. In particular, the most likely measure of effectiveness is the level of memory encoding during the branding component of the advertisement, i.e. when the brand is displayed during the advertisement.

United States Patent Nos. 4,955,938, 5,331,969 and 6,792,304 disclose techniques for obtaining a steady state visually evoked potential (SSVEP) from a subject. These patents disclose the use of Fourier analysis in order to rapidly obtain the SSVEP's and changes thereto. WO 99/59470 A1 and US 2006/184058 A1 show the calculation of amplitude and phase changes of SSVEP.

Reference is made to articles entitled Brain-Imaging Detection of Visual Scene Encoding in Long-Term Memory for TV Commercials, John R. Rossiter and Richard B. Silberstein, March/April 2001 Journal of Advertising Research, Pages 13-21 *and* Frontal Steady-State Potential Changes Predict Long-Term Recognition Memory Performance by Richard B. Silberstein, Philip G. Harris, Geoffrey A. Nield and Andrew Pipingas, International Journal of Psycho Physiology 39 (2000) 79-85*,* the content of these articles are incorporated herein by cross-reference. The first article describes an experiment using steady state probe topography (SSPT), which is a technique that involves presenting a visual or auditory or combined task such as an advertisement whilst subjects view a peripheral flickering light. In the experiment, subjects were tested for long term memory recall of static frames from TV commercials presented to the subjects one week after viewing the commercials. The article suggests that recall of static frames from the commercial by subjects provides a measure of evaluation of the success of the TV commercial.

It has now been appreciated that the effectiveness of commercial communications or advertising can be evaluated by analysis of responses in different regions of the brain so as to identify predetermined psychological states elicited by the participants as they perceive the commercial communication or advertisement.

In this specification the expression "predetermined psychological states" includes the following:

visual attention to detail;
visual attention to global features;
multi-modal attention to detail or desirability;
multi-modal attention to global features or desirability;
emotional intensity;
attraction-repulsion;
engagement; and
behavioural intent.

The expression does not include long term memory encoding per se because long term memory encoding is not considered to be a psychological state for the purposes of the methods of assessment described herein.

SUMMARY

The invention is set out in the independent claims.

According to the present disclosure there is provided a method of quantitatively assessing the effectiveness of an audio visual, visual or audio advertisement including the steps of:

presenting the advertisement to a plurality of subjects, the advertisement having a sequence of audio visual, visual and/or audio features which occur as a function of time; obtaining, during presentation of the advertisement, EEG signals from the subjects from predetermined scalp sites thereof;
calculating SSVEP amplitudes and/or phase differences from EEG signals obtained from said predetermined scalp sites in order to obtain output signals which represent predetermined psychological states of each subject to said features as a function of time;
combining the output signals from said subjects to obtain pooled output signals; and
displaying the pooled output signals to thereby enable quantitative assessment of the subjects' responses to said features of the advertisement in order to assess the effectiveness of the features of the advertisement.

The disclosure also provides a system for quantitatively assessing the effectiveness of an audio visual, visual or audio advertisement including:

display means for presenting the advertisement to a plurality of subjects, the advertisement having a sequence of audio visual, visual or audio features which occur as a function of time;
means for obtaining, during presentation of the advertisement, EEG signals from said at least one subject from predetermined scalp sites of said subjects; and
means for calculating SSVEP amplitudes and/or phase differences from signals obtained from the predetermined sites in order to obtain output signals which represent said predetermined psychological states of said at least one subject to said features as a function of time, to thereby enable quantitative assessment of said subjects' responses to said features of the advertisement in order to assess the effectiveness of the features of the advertisement.

The disclosure also provides a method of measuring steady-state visually evoked potential (SSVEP) of a subject including the step of applying time varying flicker signals only to the peripheral vision regions of the retina of a subject and not applying said time varying flicker signals to the centre of vision (fovea) of the subject.

DETAILED DESCRIPTION

The disclosure will now be further described with reference to the accompanying drawings, in which:

FIGURE 1 is a schematic diagram of a system of the disclosure;
FIGURE 2 is a schematic view showing in more detail the manner in which visual flicker stimuli are presented to a subject;
FIGURE 3 is a schematic view illustrating the International 10-20 System of electrode locations;
FIGURE 4 is a diagrammatic representation showing opacity as a function of radius of a screen which is used in the system of the disclosure;
FIGURE 5 represents still frames from a video advertisement; and
FIGURES 6 to 11 illustrate data obtained from subjects as a function of time.

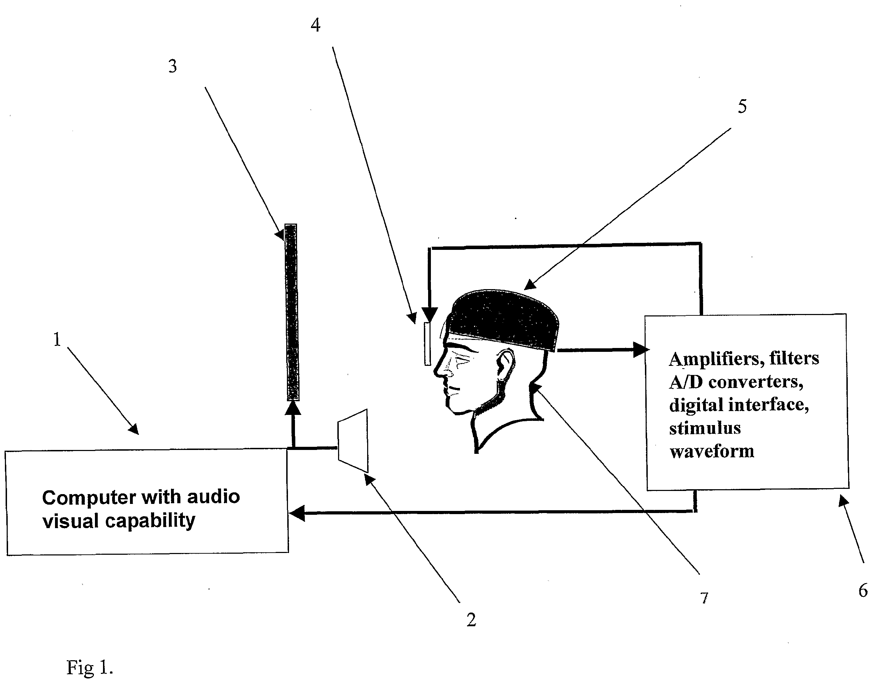

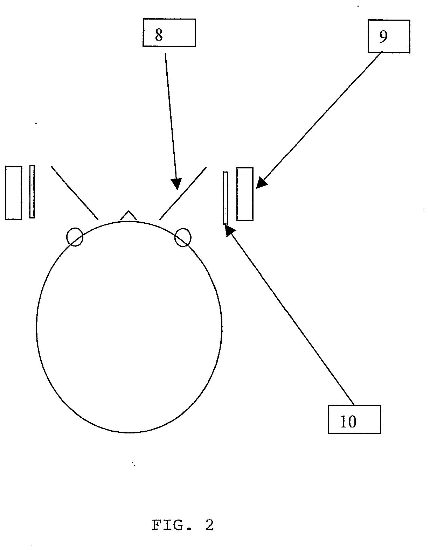

Figure 1 schematically illustrates a system for determining the response of a subject or a group of subjects to audiovisual material presented on a video screen 3 and loudspeaker 2. The system includes a computer 1 which controls various parts of the hardware and also performs computation on signals derived from the brain activity of the subject 7, as will be described below. The computer 1 also holds the television program and advertisements which can be presented to one or more subjects 7 on the screen 3 and/or through the loudspeaker 2.
The subject or subjects 7 to be tested are fitted with a headset 5 which includes a plurality of electrodes for obtaining brain electrical activity from various sights on the scalp of the subject 7. The headset includes a visor 4 which has for the left and right eyes of the subject half silvered mirrors 8 and white light Light Emitting Diode (LED) arrays 9, as shown in Figure 2. The half silvered mirrors 8 are arranged to direct light from the LED arrays 9 towards the eyes of the subject 7. The LED arrays 9 are controlled so that the light intensity therefrom varies sinusoidally as a function of time, under the control of control circuitry 6. The control circuitry 6 includes a waveform generator for generating the sinusoidal signal. The circuitry 6 also includes amplifiers, filters, analogue to digital converters and a Universal Serial Bus (USB) interface or a Transmission Control Protocol (TCP) interface or other digital interface for coupling the various electrode signals into the computer 1, as will be described in more detail below.
A translucent screen 10 is located in front of each LED array 9. Printed on the screen is an opaque pattern. The opacity of the opaque pattern is a maximum in a circular area in the centre of the centre of the screen. Beyond the circular area, the opacity falls off smoothly with radial distance from the circular area circumference, preferably, the opacity falls off as a Gaussian function described by Equation 1. The screen therefore reduces the flicker in the central visual field thus giving subjects a clear view of the visually presented material. The size of the central opaque circle (not shown) should be such as to occlude the visual flicker in the central visual field between 4°-6° vertically and horizontally. In other words the screen 10 blocks the flicker from the fovea of the subjects. The screen 3 presenting the advertisements typically subtends an angle of 10°-14° vertically and horizontally as measured from the eyes of the subject.

If r<$R$ then $P$=1
If r≥$R$ then $P$ is given by the equation 1 below.

$$P = e^{-(r-R)^2/G^2} \qquad \text{Equation 1}$$

where P is the opacity of the pattern on the translucent screen 10

An opacity of $P$=1.0 corresponds to no light being transmitted through the screen while an opacity of $P$=0 corresponds to complete transparency.

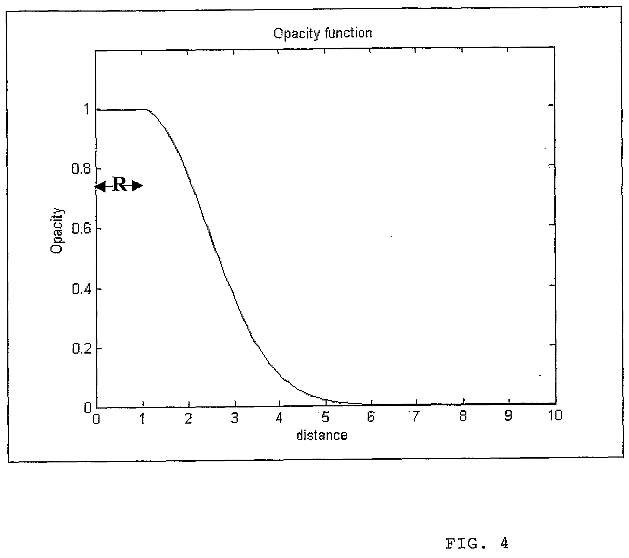

R is the radius of the central opaque disk while r is the radial distance from the centre of the opaque disk. $G$ is a parameter that determines the rate of fall-off of opacity with radial distance. Typically $G$ has values between $R$/4 and 2$R$. Figure 4 illustrates the fall-off of opacity with radial distance from the centre of the disk. In Figure 4, $R$=1 and $G$=2R. While a Gaussian fall-off of opacity with radius is preferable, any function that is smooth and has a zero gradient at r=$R$ and at r>3$G$ will be suitable.

The computer 1 includes software which calculates SSVEP amplitude phase and/or coherence from each of the electrodes in the headset 5. Details of the hardware and software required for generating SSVEP are well known and need not be described in detail. In this respect reference is made to the aforementioned United States patent specifications which disclose details of the hardware and techniques for computation of SSVEP. Briefly, the subject 7 views the video screen 3 through the special visor 4 which delivers a background flicker to the peripheral vision. The frequency of the background flicker is typically 13Hz but may be selected to be between 3Hz and 50Hz. More than one flicker frequency can be presented simultaneously. The number of frequencies can vary between 1 and 5. Brain electrical activity will be recorded using specialized electronic hardware that filters and amplifies the signal, digitizes it in the circuitry 6 where it is then transferred to the computer 1 for storage and analysis. SSPT is also used to ascertain regional brain activity at the scalp sites using SSPT analysis software, which is known and does not need to be described herein.

As mentioned above, the visor 4 includes LED arrays 9. In one embodiment, the light therefrom is varied sinusoidally. An alternative approach utilises pulse width modulation where the light emitting sources are driven by 1-10Khz pulses and the pulse duration is proportional to the brightness of the light emitting sources. In this embodiment, the control circuitry 6 receives a digital input stream from the computer 1 and outputs pulse width modulated pulses at a frequency of 1-10Khz. The time of each positive going zero-crossing from the sinusoidal stimulus waveform or combination of stimulus waveforms is preferably determined to an accuracy of about 10 microseconds and stored in the memory of the computer 1.

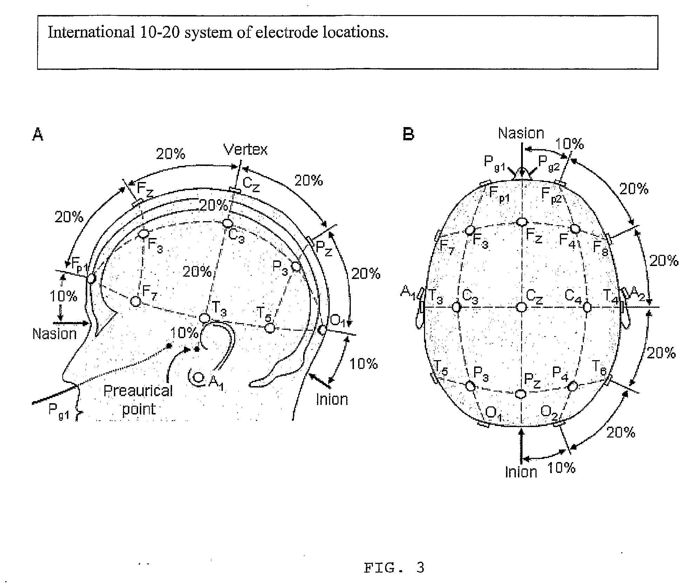

Brain electrical activity is recorded using outputs from the multiple electrodes in headset 5 or another commercially available multi-electrode system such as Electro-cap (ECI Inc., Eaton, Ohio USA). The number of electrodes is normally not less than 8 and normally not more than 128, typically 16 to 32. The electrodes are disposed so as to obtain outputs from selected scalp sites which are identified by the International 10-20 System of electrode locations shown schematically in Figure 3.

Brain activity at each of the electrodes is conducted to the control circuitry 6. The circuitry 6 includes multistage fixed gain amplification, band pass filtering and sample- and-hold circuitry for each channel. Amplified/filtered brain activity is preferably digitized to 16-24 bit accuracy at a rate not less than 300Hz and transferred to the computer 1 for storage on hard disk (not shown). The timing of each brain electrical sample together with the time of presentation of different features of the audiovisual material are also registered and preferably stored to an accuracy of 10 microseconds. The details of the control circuitry 6 are well known and need not be described.

**SSVEP amplitude and phase**

The digitized brain electrical activity (electroencephalogram or EEG) together with timing of the stimulus zero crossings enables one to calculate the SSVEP elicited by the flicker at a particular stimulus frequency from the recorded EEG or from EEG data that has been pre-processed using Independent Components Analysis (ICA) to remove artefacts and increase the signal to noise ratio. ICA is a mathematical technique for decomposing a signal into independent constituent components. This can be used to remove artefacts because artefact signals will be independent of brain activity. Bell A.J. and Sejnowski T.J. 1995, An information maximisation approach to blind separation and blind deconvolution, Neural Computation, 7, 6, 1129-1159; T-P. Jung, S. Makeig, M. Westerfield, J. Towhsehd, E. Courcheshe and T.J. Sejnowskik, Independent components analysis of single-trial event-related potential Human Brain Mapping, 14(3):168-85,2001.

## Calculation of SSVEP amplitude and phase

Calculation of SSVEP amplitude and phase coefficients for each stimulus cycle for a given stimulus frequency can be accomplished used Fourier techniques using Equations 2 and 3 below.

$$a_n = \frac{1}{S\Delta\tau} \sum_{i=0}^{S-1} f(nT + i\Delta\tau) \cos\left( \frac{2\pi}{T}(nT + i\Delta\tau) \right)$$

$$b_n = \frac{1}{S\Delta\tau} \sum_{i=0}^{S-1} f(nT + i\Delta\tau) \sin\left( \frac{2\pi}{T}(nT + i\Delta\tau) \right)$$

Equation 2

Equation 2 describes the calculation of stimulus frequency Fourier coefficients for a single cycle of the flicker stimulus. Essentially, the EEG output is sampled at specific points in time and is represented by $f(nT+i\Delta\tau)$ while the stimulus is represented by the cosine function in Equation 2 and the stimulus waveform shifted by 90 degrees is represented by the sine function. Equation 2 enables calculation of the single cycle Fourier coefficients (real and imaginary) by calculating the sum of the product of the EEG output waveform and the stimulus waveform (cos) and the sum of the product of the EEG output waveform and the stimulus waveform phase shifted by 90 degrees (sin) over a single cycle. The single cycle Fourier coefficients are smoothed by averaging overlapping sets of Fourier coefficients, where:

$a_n$ and $b_n$ are the cosine and sine Fourier coefficients respectively where;
n represents the nth stimulus cycle;
S is the number of samples per stimulus cycle (16);
$\Delta\tau$ is the time interval between samples;
T is the period of one cycle; and
$f(nT+i\Delta\tau)$ is the EEG signal (raw or pre-processed using ICA).

$SSVEP_{amplitude}$ and $SSVEP_{phase}$ can be calculated using Equation 3 below.

$$SSVEP_{amplitude} = \sqrt{\left( A_n^2 + B_n^2 \right)}$$

$$SSVEP_{phase} = a\tan\left( \frac{B_n}{A_n} \right)$$

Equation 3

Where $A_n$ and $B_n$ are overlapping smoothed Fourier coefficients calculated by using Equation 4 below.

$$A_n = \sum_{i=1}^{i=N} a_{n+i} \Big/ N$$

$$B_n = \sum_{i=1}^{i=N} b_{n+i} \Big/ N$$

Equation 4

Amplitude and phase components can be calculated using either single cycle Fourier coefficients ($a_n$ and $b_n$) or coefficients that have been calculated by smoothing across multiple cycles ($A_n$ and $B_n$).

Equations 3 and 4 describe the procedure for calculating the smoothed SSVEP coefficients for a single subject. For pooled data, the SSVEP coefficients ($A_n$ and $B_n$) for a given electrode are averaged (or pooled) across all of the subjects or a selected group of subjects.

As the number of cycles used in the smoothing increases, the signal to noise ratio increases while the temporal resolution decreases. The number of cycles used in the smoothing is typically in excess of 5 and less than 130.

The above equations apply to scalp recorded data as well as brain electrical activity inferred at the cortical surface adjacent to the skull and deeper regions. Activity in deeper regions of the brain such as the orbito-frontal cortex or ventro-medial cortex can be determined using a number of available inverse mapping techniques such as BESA, EMSE and LORETA.

Once the system has been set up and headsets 5 fitted to one or more subjects, the audiovisual program or advertisement to be evaluated is displayed on the screen 3, the visual flicker is switched on in the visor 4 and brain electrical activity of the subject or subjects is recorded continuously on the computer 1. At the end of the recording stage, the SSVEP amplitude and phase are separately calculated for each subject. Once all recordings are completed, group averaged data is calculated by averaging the smoothed SSVEP amplitude and phase data from selected subjects to be included in the group (eg male, female, young, old). Changes in regional synaptic excitation or inhibition are indicated by SSVEP phase changes while changes in regional activity (irrespective of whether these changes are associated with excitation or inhibition) are indicated by changes in SSVEP amplitude. Typically, such inverse mapping techniques require 19 or more scalp recording sites and preferably 64 or more scalp recording sites.

In accordance with the invention, cognitive and emotional measures at specific points in time during the advertisement can be derived from the pattern of SSVEP phase and SSVEP amplitude changes at the various scalp recording sites or inferred brain regional activity. Scalp recording sites typically used are identified by reference to the International 10-20 System (Jasper HH: Electroenceph. Clin. Neurophysiol. 1958; 10: 370-1) shown in Figure 3. The psychological states associated with SSVEP phase and amplitude changes at scalp and brain cortical locations are summarised below.

Activity at various brain sites can be determined using a variety of possible inverse mapping techniques such as EMSE and LORETA that express brain activity in selected brain regions in terms of a function of SSVEP amplitude and phase measures recorded over the entire scalp. Typically such techniques require more than 20 scalp recording sites and preferably 64 or more scalp recording sites.

### 1. *Visual Attention to Detail*

This measure refers to the level of visual attention to detail. Examples include visual attention to text and numbers as well as visual attention to details of a scene or a face.

SSVEP phase advance or amplitude change at the left occipital region, preferably electrode $O_1$ has been found to be relevant to assessment of the subject's *Visual Attention to Detail.* If inverse mapping techniques are used, the relevant location in the left cerebral cortex is the vicinity of Brodmans area 17.

### 2. *Visual Attention to Global Features*

This measure refers to the level of visual attention to global features. Examples include visual attention to images such as scenery or faces as a whole.

SSVEP phase advance or amplitude change at the right occipital region, preferably electrode $O_2$ has been found to be relevant to the assessment of the subject's *Visual Attention* to *Global Features* including responses to facial expressions displayed on the screen. If inverse mapping techniques are used, the relevant location in the right cerebral cortex is the vicinity of Brodmans area 17.

### 3. *Multi-modal Attention to Detail or Desirability*

This measure refers to multi-modal or multi-sensory attention. Typically this includes both auditory and visual attention to detail in the visual domain or speech in the auditory domain. When this attention measure is also associated with objects, it indexes the level of desirability associated with the said object.

SSVEP phase advance or amplitude change at the left parietal region, preferably electrode $P_3$ has been found to be relevant to the assessment of the subject's *Multi-modal Attention to Detail or Desire* for the subject matter of the screened advertisement. If inverse mapping techniques are used, the relevant location in the left cerebral cortex is the vicinity of the left intraparietal area.

### 4. *Multi-modal Attention to Global Features or Desirability*

This measure refers to multi-modal or multi-sensory attention. Typically this includes both auditory and visual attention to global features such as facial expression, scenery in the visual domain or music in the auditory domain. When this attention measure is also associated with objects, it indexes the level of desirability associated with the said object.

SSVEP phase advance or amplitude change at the right parietal region, preferably electrode $P_4$ has been found to be relevant to the assessment of the subject's *Multi-modal Attention to Global Features or Desire* for the subject matter of the screened advertisement. If inverse mapping techniques are used, the relevant location in the left cerebral cortex is the vicinity of the right intraparietal area.

### 5. *Emotional Intensity*

This measure indicates the intensity of the emotional state experienced by subjects. This measure is independent of the specific emotion such as joy, fear, anger, anxiety, etc.

SSVEP phase advance or amplitude change at the right parieto-temporal region, preferably from a single electrode which is approximately equidistant from right hemisphere electrodes $O_2$, $P_4$ and $T_6$, has been found to be relevant to the assessment of the subject's *Emotional Intensity* response to different parts of the screened advertisement. If inverse mapping techniques are used, the relevant location in the right cerebral cortex is the vicinity of the right parieto-temporal junction.

### 6. *Attraction-Repulsion sometimes termed Like-Dislike)*

This measure indicates the extent to which subjects are attracted or repelled to characters or situations represented in the advertisements. When activity at the combination of left hemisphere sites is greater than at the combination of right hemisphere sites, subjects are attracted to the scene or character and *vice versa.*

The difference between SSVEP phase advance at the left frontal/prefrontal and right frontal/prefrontal regions has been found to be relevant to the assessment of the subject's *Attraction-Repulsion* response to the screened advertisement. *Attraction* is indicated by a larger phase advance in the left hemisphere compared to the right while *Repulsion* is indicated by a larger phase advance in the right hemisphere compared to the left. *Attraction-Repulsion* can be calculated as follows:

$$Attraction = (a_1 * \text{ SSVEP phase advance at electrode } F_3 + a_2 * \text{ SSVEP phase advance at electrode } F_{p1} - a_3 * \text{ SSVEP phase advance at electrode } F_4 - a_4 * \text{ SSVEP phase advance at electrode } F_{p2})$$

$$\text{where } a_1 = a_2 = a_3 = a_4 = 1.0 \qquad \text{Equation 5}$$

A positive value for the *Attraction* measure is associated with the subjects finding the screened material attractive and liked while a negative measure for *Attraction* is associated with repulsion or dislike of the screened material.

If inverse mapping techniques are used, the *Attraction* can be calculated as follows:

$$\text{Attraction} = (c_1 * \text{right orbito-frontal cortex (in vicinity of Brodman area 11)}$$
$$+ c_2 * \text{right dorso-lateral prefrontal cortex (in vicinity of Brodman area 9)} + c_3 * \text{left}$$
$$\text{orbito frontal cortex (in vicinity of Brodman area 11)} + c_4 * \text{left dorso-lateral}$$
$$\text{prefrontal cortex (vicinity of Brodman area 9))}$$

where $c_1 = 1$, $c_2 = 1$, $c_3 = 1$, $c_4 = 1$        Equation 6

and wherein the averaged SSVEP phase measures at the above sites are inserted in Equation 6.

### 7. *Engagement*

This measure indicates the level of Engagement. Engagement refers to the extent that different components of the advertisement elicit a sense of personal relevance.

*Engagement* of the subject in the screened advertisement can be calculated by the weighted mean SSVEP phase advance at prefrontal sites described by the expression below.

$$\textit{Engagement} = (b_1 * \text{SSVEP phase advance at electrode } F_3 + b_2 * \text{SSVEP phase}$$
$$\text{advance at electrode } P_{p1} + b_3 * \text{SSVEP phase advance at electrode } F_4 + b_4 *$$
$$\text{SSVEP phase advance at Electrode } F_{p2})$$

where $b_1 = 0.1$, $b_2 = 0.4$, $b_3 = 0.1$, $b_4 = 0.4$        Equation 7

If inverse mapping techniques are used, the relevant expression is:

$$\textit{Engagement} = (d_1 * \text{right orbito frontal cortex (in vicinity of Brodman area 11 )} +$$
$$d_2 * \text{right dorso-lateral prefrontal cortex (in vicinity of Brodman area 9)} + d_3 * \text{left}$$
$$\text{orbito frontal cortex (in vicinity of Brodman area 11)} + d_4 * \text{left dorso-lateral}$$
$$\text{prefrontal cortex (in vicinity of Brodman area 9))}$$

where $d_1 = 0.1$, $d_2 = 0.4$, $d_3 = 0.1$, $d_4 = 0.4$        Equation 8

and wherein the averaged SSVEP phase measures at the above sites are inserted in Equation 8.

### 8. *Behavioural Intent*

*Behavioural Intent* or the likelihood that the subjects are likely to act in accordance with the purpose of the screened advertisement, that is make a purchase, is associated with memory encoding at the time of branding. This is indicated by the level of long term memory encoding at the time that the brand is portrayed in the advertisement. Long term memory encoding can be assessed as follows:

SSVEP phase advance or amplitude change at left frontal region, preferably a single electrode which is approximately equidistant from left hemisphere electrodes $C_3$, $F_3$ and $F_7$, has been found to be relevant to the assessment of the subject's long term memory encoding for details and verbal memories of the subject matter of the screened advertisement. If inverse mapping techniques are used, the relevant location in the left cerebral cortex is the vicinity of Brodmans areas 6, 44, 45, 46 and 47.

SSVEP phase advance or amplitude change at right frontal region, preferably approximately equidistant from right hemisphere electrodes $C_4$, $F_4$ and $F_8$ has been found to be relevant to the assessment of the subject's long term memory encoding for emotional and non-verbal memories of the subject matter of the screened advertisement. If inverse mapping techniques are used, the relevant location in the right cerebral cortex is the vicinity of Brodmans areas 6, 44, 45, 46 and 47.

Once data from long term memory encoding has been obtained for verbal and/or non-verbal memories, this can be correlated with the time sequence of the advertising material being presented to determine the relevant levels of memory encoding when the brand is portrayed in the advertisement. After this correlation, it is then possible to assess whether or not the subjects are likely to purchase goods or services shown in the advertisement.

**Example**

The following procedure is used to evaluate a television advertisement to a client such as the brand manager of a company and/or advertising agency.

A selected number of subjects, say 50, are seated in a test room and the headsets 5 are placed on their heads. The visors 4 are then placed in position and adjusted so that the foveal block by the screens 10 prevents the appearance of the flicker over the screens 3 where the advertisements are presented. The number of subjects in a recording session is variable and typically can vary from 1 to over 100. When pooling subjects to create the average response, the number of subjects whose data is to be included in the average should be no less than 16.

The advertisements to be tested were included in an 'advertising break' comprising a block of 3 to 6 advertisements incorporated in a television program to simulate a standard commercial TV viewing environment. Each advertising break is followed immediately by a 30 second sequence of still images of scenery and a musical accompaniment. Typically, 60 images were presented over the period of 30 seconds with each image present for 0.5 seconds. The same sequence of images and music were presented after each advertisement break. Brain activity levels during the adjacent scene images are used as a reference level for brain activity during the preceding advertisement break. This enables removal of any long-term changes in brain activity that may occur over the time course of the recording period.

Pooled or averaged data at various brain sites can then be displayed to the client as the difference between the reference level and the value at other points in time during the advertisement. A fixed offset between 0.2 to 0.6 preferably 0.3 radians is then added to the abovementioned difference to yield the SSVEP phase data at each scalp site.

To minimize subject irritation or discomfort to the subject due to the flicker, the flicker stimulus is of variable intensity and only switched to the highest intensity when material of interest to the client such as the block of advertisements is present on the screen. During the periods that material of interest is not present on the screen, the stimulus intensity is typically zero and never more than 10% of the typical value used when material of interest is on the screen. Preferably, the stimulus is not switched on abruptly but is slowly increased before the advertisement break and decreased slowly after the end of an advertisement break. Typically, the stimulus is increased linearly over a 30-60 second epoch prior to the advertisement break so that it reaches its maximum value 60 seconds prior to the first advertisement. Immediately the sequence of reference images at the end of the advertisement break ends, the stimulus intensity is linearly reduced to the minimum value over a 30 second period. The slow linear increase and decrease of stimulus intensity occurs for every advertisement break.

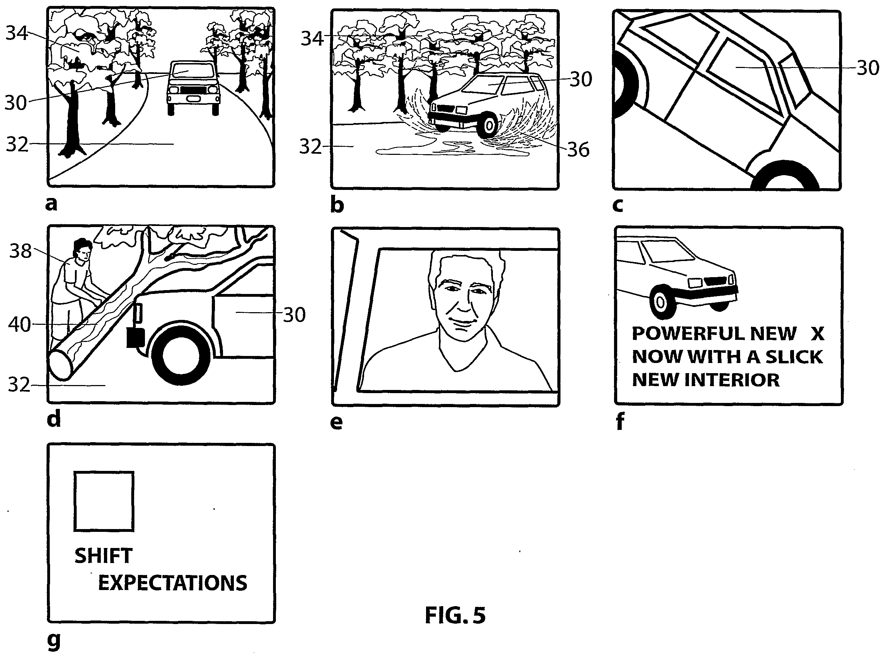

Figure 5 schematically illustrates the television advertisement to be assessed. The advertisement is in relation to a sports utility vehicle 30. The vehicle 30 is being driven along a road 32 in a dark forest 34 on a wet and stormy night, as indicated by still segment a. The vehicle 30 drives through a partially flooded roadway so as to cause a large spray 36 from the vehicle, as indicated in still segment b. The vehicle is then shown traversing steep grade, as shown in still segment c. The driver 38 then stops the vehicle where a large log 40 has fallen on the road 32 so as to remove it, as shown in still segment d. After clearing the branch from the road, the driver is about to re-enter the car when he realises that he is covered with mud and would soil the interior of the car were he to enter immediately, as indicated in still segment e. This scene features an abrupt change in the soundtrack when the music stops and is replaced by a period of silence. The driver ruefully finds a cake of soap in the glove compartment and proceeds to take a bath in the nearby stream during the storm. The final segment of the animated part of the advertisement shows a block of text with the brand of the vehicle in the text block, as indicated by still segment f. The final segment g runs for about 2 seconds and shows the brand and product being advertised.

Once the final subject recording has taken place, individual SSVEP data is pooled for each specific group of subjects, for example, separate pooling or averaging could be effected for the entire group and also for subgroups such as male and female and young and old. To display the SSVEP phase data in relationship to the associated commercial, an in-house program (NV-Show) is used. NV-Show displays the advertisement in a portion of a computer video monitor and the associated SSVEP phase data as a graph below. As the advertisement is played in the upper portion of the screen, the graph describing the SSVEP phase data is revealed below. Alternatively, the graph of SSVEP phase data can be present throughout the advertisement playing time and a moving time marker used to indicate the SSVEP phase data corresponding to time in the advertisement being displayed.

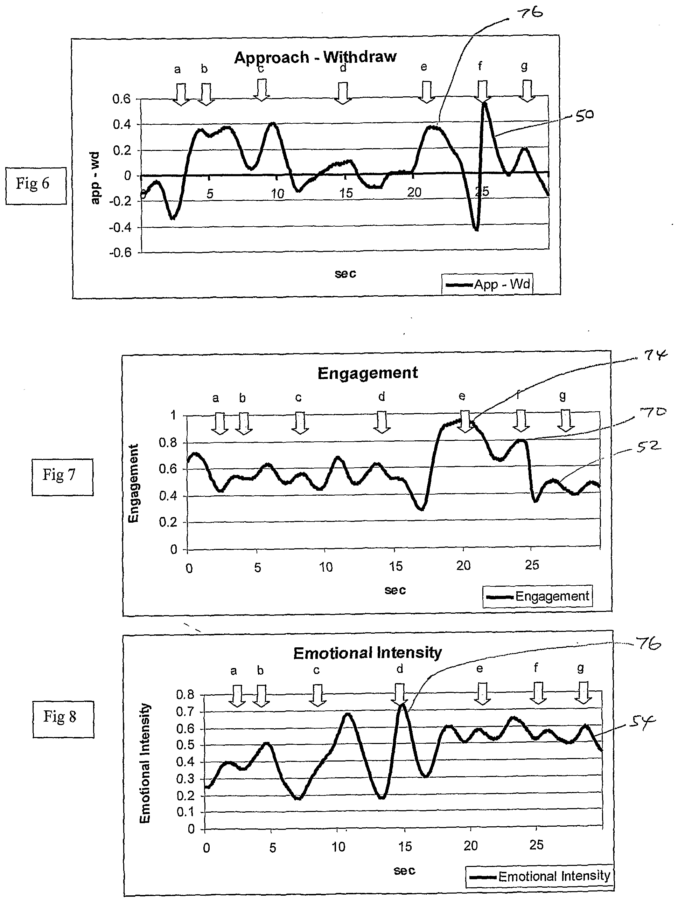

Figures 6 to 11 illustrate the brain measurement values at the various brain sites during the advertisement. The block arrows labelled 'a' to 'g' in Figures 6 to 11 correspond to the points in time a to g shown in Figure 5.

In Figure 6, the line 50 shows the *Attraction-Repulsion* measure during the advertisement. The *Attraction-Repulsion* measures are calculated using Equation 5 above and the results are displayed in graphical form on a time scale which corresponds to the duration of the advertisement. Positive values or values above the horizontal time axis correspond to 'approach' or 'like' while negative values correspond to points in time where the audience 'withdraws' or 'dislikes'. For the approach-withdraw measure in this study, any value larger than =.3 or smaller than -0.3 is statistically significant.

If a greater number of electrodes were used, it would be possible to use inverse mapping techniques to obtain somewhat higher resolution in the data and by using Equation 5.

In Figure 7 the line 52 indicates the level of audience *Engagement* in the advertisement as a function of time. The data is calculated using Equation 7 and is again presented graphically on a time scale which corresponds to that of the advertisement. The term *Engagement* relates to the level of personal or emotional relevance experienced by the subjects. In the study illustrated, *Engagement* must exceed 0.4 to be significantly greater than baseline level while an engagement level above 0.7 is considered high.

If inverse mapping techniques are used, Equation 8 can be used to calculate the measure for *Engagement.*

In Figure 8 the line 54 indicates the equivalent changes in *Emotional Intensity* elicited by the advertisement. *Emotional Intensity* refers to the intensity of emotion experienced by the subjects irrespective of the type of emotion, e.g. fear, joy, anger, excitement etc. The data can be calculated as described above by using SSVEP phase advance or amplitude change at right parieto-temporal region, from an electrode which is approximately equidistant from the right hemisphere electrodes $O_2$, $P_4$ and $T_6$. If inverse mapping techniques are used, the relevant location in the right cerebral cortex is in the vicinity of the right parieto-temporal junction. Once again, any level above 0.4 is significantly higher than background while a level above 0.7 is considered high.

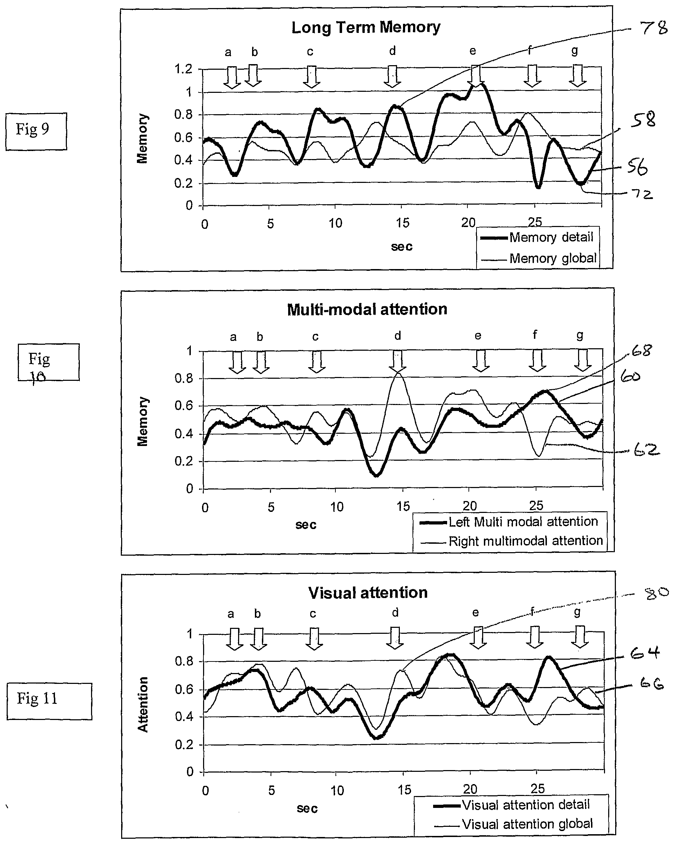

The lines 56 and 58 in Figure 9 indicate memory encoding in detail and memory encoding global respectively during the advertisement. This measure indicates how effectively different components of the advertisement are being stored in long term memory. This measure is especially important as an advertisement is only effective if the key messages and information about the brand enters long-term memory. It has been found that the likely impact of the advertisement on *Behavioural Intent* is positively correlated with the level of long-term memory encoding during the time that branding information is presented in the advertisement. Thus *Behavioural Intent* or the likely impact on behaviour of the subjects is indicated by the level of memory encoding at the time that branding information is presented in the advertisement, i.e. at points f and g where the branding information is positively displayed during the advertisement. Both memory traces are important in regard to *Behavioural Intent.* As long as either left or right memory encoding state is high during "branding", *Behavioural Invent* or the propensity to act on the message of the advertisement, is high.

In Figure 10 the lines 60 and 62 indicate left hemisphere multi-modal attention and right hemisphere multi-modal attention respectively. Multi-modal attention includes visual and auditory attention to details of the advertisement being displayed to the subjects. Specifically, the line 60 indicates attention to detailed features such as text and speech and the line 62 indicates attention to global features such as facial expression and music (for example).

In Figure 11 the lines 64 and 66 indicate the visual attention to detail and visual attention globally of the subjects to the advertising material being displayed. Specifically, the line 64 indicates visual attention to detailed features such as text. The line 64 indicates visual attention to global features such as facial expression (for example).

## Assessment

For this advertisement to be assessed as effective, it is necessary for memory encoding to be high during the times when the product or product benefits are emphasized, such as at point f and when the brand is explicitly featured such as at point g. As can be seen in Figure 10, left memory encoding is moderate to high during point f as indicated by peak 68 in the line 60 indicating that there is adequate memory encoding for the text information THE POWERFUL NEW X; NOW WITH A SLICK NEW INTERIOR near the end of the advertisement. During this point in time, *Engagement* is also high indicating a high degree of personal relevance regarding the features mentioned as indicated by the peak 70 in line 52.

By contrast, the memory encoding for the branding information at point g is low and *Engagement* is also low for this time as indicated by trough 72 in the line 56. This indicates that the subjects will remember the vehicle interior but will not recall the brand. The *Behavioural Invent* measure is also low indicating that the advertisement is not effective as the subjects will be less inclined to act in accordance with the advertisement. This is again indicated by the trough 72.

Accordingly, the advertisement can be assessed as not being commercially effective in that the branding information is not satisfactorily encoded in long-term memory. The dramatic structure of the advertisement is, however, effective in that 'joke' at point e in the advertisement works because the scene where the driver realizes he can't re-enter the car without washing is well encoded in long-term memory and also leads to highest level of *Engagement* as indicated by the peak 74 in line 52. In addition, there is a high level of *Attraction* that is associated with this humour as indicated by peak 76 in line 50.

It is also apparent that the overall structure of the advertisement is effective in that the key dramatic scene where the driver has to get out of the car to clear the road at point d is associated with high levels of *Emotional Intensity* as indicated by the peak 76 in line 54; high levels of memory encoding as indicated by peak 78 in line 56; and a high level of *Attention to Global Features* as indicated by the peak 80 in line 66.

After assessment of the advertisement using the techniques of the invention, the client can appreciate that the overall effect of the advertisement is positive but some modification is required at points f and g where the brand is introduced. Accordingly, the client is in a position to modify the advertisement so that it should become highly successful and avoid incurring substantial advertising costs in an advertising program which does not induce viewers to purchase the product.

It will be appreciated that the principles of the invention can be applied other audiovisual commercial communications in addition to advertisements.

Many modifications will be apparent to those skilled in the art without departing from the spirit and scope of the invention.

**Claims**

1.  A method of quantitatively assessing the effectiveness of an audiovisual, visual and/or audio advertisement including the steps of:

    presenting the advertisement to a plurality of subjects, the advertisement having a sequence of audiovisual, visual and/or audio features which occur as a function of time;
    obtaining, during presentation of the advertisement, EEG signals from the subjects from predetermined scalp sites thereof;
    calculating SSVEP amplitudes and phase differences from EEG signals obtained from said predetermined scalp sites in order to obtain output signals which represent predetermined psychological states of each subject to said features as a function of time;
    applying a sinusoidally varying visual flicker stimulus to each subject during presentation of the advertisement to thereby enable calculation of Fourier coefficients from said output signals to thereby enable calculation of said SSVEP amplitudes and phase differences;
    combining the output signals from said subjects to obtain pooled output signals, wherein the step of combining the output signals includes the step of averaging output signals from each subject, wherein said SSVEP amplitudes and phase differences are calculated by the equations:

$$SSVEP_{amplitude} = \sqrt{\left(A_n^2 + B_n^2\right)}$$

$$SSVEP_{phase} = a\tan\left(\frac{B_n}{A_n}\right)$$

    wherein $A_n$ and $B_n$ are overlapping smoothed Fourier coefficients calculated by using the equation:

$$A_n = \sum_{i=1}^{i=N} a_{n+i} \Big/ N$$

$$B_n = \sum_{i=1}^{i=N} b_{n+i} \Big/ N$$

    where: $a_n$ and $b_n$ are cosine and sine Fourier coefficients calculated by the equations:

$$a_n = \frac{1}{S\Delta\tau} \sum_{i=0}^{S-1} f(nT + i\Delta\tau) \cos\left(\frac{2\pi}{T}\left(nT + i\Delta\tau\right)\right)$$

$$b_n = \frac{1}{S\Delta\tau} \sum_{i=0}^{S-1} f(nT + i\Delta\tau) \sin\left(\frac{2\pi}{T}\left(nT + i\Delta\tau\right)\right)$$

where:

$a_n$ and $b_n$ are the cosine and sine Fourier coefficients respectively where:

n represents the nth flicker stimulus cycle;
S is the number of samples per flicker stimulus cycle;
$\Delta\tau$ is the time interval between samples;
T is the period of one cycle; and
$f(nT+i\Delta\tau)$ is the EEG signal (raw or pre-processed using ICA) obtained from said predetermined scalp sites; and

displaying the pooled output signals to thereby enable quantitative assessment of the subjects' responses to said features of the advertisement in order to assess the effectiveness of the features of the advertisement;

**characterised by**:

applying electrodes to the scalp of each subject at $F_3$, $F_4$, $Pp_1$ and $Fp_2$ sites, calculating SSVEP amplitudes and phase differences from said electrodes, calculating values for engagement in features of the advertisement by a weighted mean SSVEP phase advance at said sites using the equation:

$$\text{engagement} = (b_1 * \text{SSVEP phase advance at electrode } F_3 + b_2 * \text{SSVEP}$$
$$\text{phase advance at electrode } P_{p1} + b_3 * \text{SSVEP phase advance at electrode } F_4$$
$$+ b_4 * \text{SSVEP phase advance at Electrode } F_{p2})$$

where $b_1$ = 0.1, $b_2$ = 0.4, $b_3$=0.1, $b_4$=0.4,
whereby said values indicate each subject's engagement in features of the advertisement; and/or

applying an electrode to the scalp of each subject at the $F_3$, $F_4$, $F_{p1}$ and $F_{p2}$ sites, calculating SSVEP amplitudes and phase differences from EEG signals from said electrodes, calculating values for attraction-repulsion using the equation:

$$\text{attraction} = (a_1 * \text{SSVEP phase advance at electrode } F_3 + a_2 * \text{SSVEP phase}$$
$$\text{advance at electrode } F_{p1} - a_3 * \text{SSVEP phase advance at electrode } F_4 - a_4 *$$
$$\text{SSVEP phase advance at electrode } F_{p2})$$

where $a_1$ = $a_2$ = $a_3$ = $a_4$ = 1.0
whereby said values indicate each subject's attraction or repulsion towards features of the advertisement.

2. A method as claimed in claim 1 including the step of simultaneously displaying the advertisement to said plurality of subjects.

3. A method as claimed in claim 2 including the step of selecting scalp sites in order to obtain output signals which enable assessment of:

visual attention to detail;
visual attention to global features;

multi-modal attention to detail or desirability;
multi-modal attention to global features or desirability;
emotional intensity associated;
attraction-repulsion;
engagement; or
behavioural intent
in relation to the features of the advertisement.

4. A method as claimed in claim 1 including the steps of:

obtaining SSVEP amplitude and phase from a plurality of scalp sites of each subject; and
utilising inverse mapping techniques such as BESA, EMSA or LORETA to produce modified EEG signals which represent activity in deeper regions of the brain of each subject such as the orbito-frontal cortex or the ventro-medial cortex.

5. A method as claimed in claim 1 or 4 including the step of averaging the Fourier coefficients An and $B_n$ for a selected group of subjects and then calculating the SSVEP amplitudes and SSVEP phase differences for said group of subject.

6. A method as claimed in any one of claims 1 to 5 wherein the flicker signal is applied only to the peripheral vision of each subject.

7. A method as claimed in claim 6 including the steps of directing the light towards the eyes of each subject via first and second screens so that the light passing through the screen constitutes said flicker signal and wherein each screen includes an opaque area, and wherein the method further includes the step of positioning the screens to the relative position of each subject such that said opaque areas prevent said flicker signal impinging on the fovea of each eye of each subject.

8. A method as claimed in claim 7 wherein the opacity of each screen decreases as a function of distance from its opaque area so that the intensity of the flicker signal impinging on each retina of each subject decreases in value from the central vision to the peripheral vision.

9. A method as claimed in claim 8 including the step of applying a masking pattern to each screen to define the opacity thereof, the method including the step of applying the pattern in accordance with a masking pattern function which provides zero or low gradients for changes in opacity adjacent to its opaque area and peripheral areas thereof which define parts of the flicker signal impinging on the peripheral vision of each subject.

10. A method as claimed in claim 9 wherein the opaque area of each screen is circular and wherein the masking pattern function is selected to be a Gaussian function, so that the opacity P of the screen is defined by the equation:

$$P = e^{-(r-R)^2 / G^2}$$

where:

r is the radial distance from the centre of the opaque area; and
G is a parameter that determines the rate of fall-off of opacity with radial distance, and wherein when r<R, P=1.

11. A method as claimed in claim 8 wherein G has a value in the range R/4 and 2R.

12. A method as claimed in claim 1 including the steps of applying an electrode to the scalp of each subject at the $O_1$ site, calculating SSVEP amplitudes and phase differences from EEG signals from said electrode whereby the output signals indicate each subject's visual attention to details of the advertisement.

13. A method as claimed in claim 4 including the step of utilising inverse mapping determines brain activity in the left cerebral cortex in the vicinity of Brodman's area 17 whereby the modified output signals indicate each subject's visual attention to details of the advertisement.

14. A method as claimed in claim 1 including the steps of applying an electrode to the scalp of each subject at the $O_2$ site, calculating SSVEP amplitudes and phase differences from EEG signals from said electrode whereby the output signals are indicative of each subject's visual attention to global features of the advertisement.

15. A method as claimed in claim 4 including the step of utilising inverse mapping determines brain activity in the right cerebral cortex in the vicinity of Brodman's area 17 whereby the output signals indicate each subject's visual attention to global features of the advertisement.

16. A method as claimed in claim 1 including the step of applying an electrode to the scalp of each subject at the $P_3$ site, calculating SSVEP amplitudes and phase differences from EEG signals from said electrode whereby the output signals are indicative of each subject's multi-modal attention to detail or desirability to features of the advertisement.

17. A method as claimed in claim 4 wherein the step of utilising inverse mapping determines brain activity in the left cerebral cortex in the vicinity of the intraparietal area whereby the output signals indicate each subject's multi-modal attention to detail or desirability of the features of the advertisement.

18. A method as claimed in claim 1 including the step of applying an electrode to the scalp of each subject at the $P_4$ site, calculating SSVEP amplitudes and phase differences from EEG signals from said electrode whereby the output signals indicate each subject's multi-modal attention to global features or desirability of the features of the advertisement.

19. A method as claimed in claim 4 wherein the step of utilising inverse mapping determines brain activity in the right cerebral cortex in the vicinity of the intraparietal area whereby the output signals indicate each subject's multi-modal attention to global features or desirability of the features of the advertisement.

20. A method as claimed in claim 1 including the step of applying an electrode to the scalp of each subject at a site which is approximately equidistant from sites $O_2$, $P_4$ and $T_6$, calculating SSVEP amplitudes and phase differences from EEG signals from said electrode whereby the output signals indicate each subject's emotional intensity associated with the advertisement.

21. A method as claimed in claim 4 wherein the step of utilising inverse mapping determines brain activity in the right cerebral cortex in the vicinity of the right parieto-temporal junction whereby the output signals indicate each subject's emotional intensity associated with the advertisement.

22. A method as claimed in claim 4 wherein the step of utilising inverse mapping determines brain activity in:

>   the right orbito-frontal cortex in the vicinity of Brodman area 11;
>   the right dorso-lateral prefrontal cortex in the vicinity of Brodman area 9;
>   the left orbito frontal cortex in the vicinity of Brodman area 11; and
>   the left dorso-lateral prefrontal cortex in the vicinity of Brodman area 9; and
>   calculating a value for attraction-repulsion using the equation:

$$\text{attraction} = (c_1 * \text{SSVEP phase advance at right orbito-frontal cortex (in vicinity of Brodman area 11)} + c_2 * \text{SSVEP phase advance at right dorso-lateral prefrontal cortex (in vicinity of Brodman area 9)} + c_3 * \text{SSVEP phase advance left orbito frontal cortex (in vicinity of Brodman area 11)} + c_4 * \text{SSVEP phase advance left dorso-lateral prefrontal cortex (vicinity of Brodman area 9))}$$

>   where $c_1 = 1$, $c_2 = 1$, $C_3 = 1$, $c_4 = 1$,
>   whereby said values indicate each subject's attraction or repulsion towards features of the advertisement.

23. A method as claimed in claim 4 wherein the step of utilising inverse mapping determines brain activity in:

the right orbito frontal cortex in the vicinity of Brodman area 11;
the right dorso-lateral prefrontal cortex in the vicinity of Brodman area 9;
the left frontal cortex in the vicinity of Brodman area 11; and
the left dorso-lateral prefrontal cortex in the vicinity of Brodman area 9,
calculating SSVEP amplitudes and phase differences from said modified EEG signals from said electrodes; and
calculating a value for engagement using the equation:

engagement = ($d_1$\*SSVEP phase advance at right orbito frontal cortex (in vicinity of Brodman area 11 ) + $d_2$\*SSVEP phase advance right dorso-lateral prefrontal cortex (in vicinity of Brodman area 9) + $d_3$\*SSVEP phase advance at left orbito frontal cortex (in vicinity of Brodman area 11) + $d_4$\*SSVEP phase advance at left dorso-lateral prefrontal cortex (in vicinity of Brodman area 9))

where $d_1$ = 0.1, $d_2$ = 0.4, $d_3$ =0.1, $d_4$ =0.4,
whereby said values indicate each subject's engagement in features of the advertisement.

24. A method as claimed in claim 1 including the steps of applying an electrode to the scalp of each subject at a site approximately equidistant from the $C_3$, $F_3$ and $F_7$ sites, calculating SSVEP amplitudes and phase differences from EEG signals from said electrode, whereby output signals indicate each subject's behavioural intent to the subject matter of the advertisement.

25. A method as claimed in claim 4 wherein the step of utilising inverse mapping determines brain activity in the left cerebral cortex in the vicinity of Brodman's areas 6, 44, 45, 46 and 47, calculating SSVEP amplitude and phase differences from said modified EEG signals from said electrodes whereby output signals indicate each subject's behavioural intent to the subject matter of the advertisement.

26. A method as claimed in any one of claims 1 to 25 wherein said pooled output signals are displayed graphically.

27. A method as claimed in claim 26 wherein said pooled output signals are displayed on a video monitor which simultaneously displays the advertisement being assessed.

28. A system for quantitatively assessing the effectiveness of an audiovisual, visual and/or audio advertisement including:

means for presenting the advertisement to a plurality of subjects, the advertisement having a sequence of audiovisual, visual and/or audio features which occur as a function of time;
means for obtaining, during presentation of the advertisement, EEG signals from the subjects from predetermined scalp sites thereof;
means for calculating SSVEP amplitudes and phase differences from EEG signals obtained from said predetermined scalp sites in order to obtain output signals which represent predetermined psychological states of each subject to said features as a function of time;
means for applying a sinusoidally varying visual flicker stimulus to each subject during presentation of the advertisement to thereby enable calculation of Fourier coefficients from said output signals to thereby enable calculation of said SSVEP amplitudes and phase differences;
means for combining the output signals from said subjects to obtain pooled output signals, wherein the step of combining the output signals includes the step of averaging output signals from each subject, wherein said SSVEP amplitudes and phase differences are calculated by the equations:

$$SSVEP_{amplitude} = \sqrt{\left(A_n^2 + B_n^2\right)}$$

$$SSVEP_{phase} = a\tan\left(\frac{B_n}{A_n}\right)$$

wherein An and $B_n$ are overlapping smoothed Fourier coefficients calculated by using the equation:

$$A_n = \sum_{i=1}^{i=N} a_{n+i} \Big/ N$$

$$B_n = \sum_{i=1}^{i=N} b_{n+i} \Big/ N$$

where: $a_n$ and $b_n$ are cosine and sine Fourier coefficients calculated by the equations:

$$a_n = \frac{1}{S\Delta\tau} \sum_{i=0}^{S-1} f(nT + i\Delta\tau)\cos\left(\frac{2\pi}{T}(nT + i\Delta\tau)\right)$$

$$b_n = \frac{1}{S\Delta\tau} \sum_{i=0}^{S-1} f(nT + i\Delta\tau)\sin\left(\frac{2\pi}{T}(nT + i\Delta\tau)\right)$$

where:

$a_n$ and $b_n$ are the cosine and sine Fourier coefficients respectively where:

n represents the nth flicker stimulus cycle;
S is the number of samples per flicker stimulus cycle;
$\Delta\tau$ is the time interval between samples;
T is the period of one cycle; and
$f(nT+i\Delta\tau)$ is the EEG signal (raw or pre-processed using ICA) obtained from said predetermined scalp sites; and

means for displaying the pooled output signals to thereby enable quantitative assessment of the subjects' responses to said features of the advertisement in order to assess the effectiveness of the features of the advertisement;

**characterised by**:

means for applying electrodes to the scalp of each subject at $F_3$, $F_4$, $P_{p1}$ and $F_{p2}$ sites, calculating SSVEP amplitudes and phase differences from said electrodes, calculating values for engagement in features of the advertisement by a weighted mean SSVEP phase advance at said sites using the equation:

engagement = ($b_1$ * SSVEP phase advance at electrode $F_3$ + $b_2$ * SSVEP phase advance at electrode $P_{p1}$ + $b_3$ * SSVEP phase advance at electrode $F_4$ + $b_4$ * SSVEP phase advance at Electrode $F_{p2}$)

where $b_1 = 0.1$, $b_2 = 0.4$, $b_3 = 0.1$, $b_4 = 0.4$,
whereby said values indicate each subject's engagement in features of the advertisement; and/or

means for applying an electrode to the scalp of each subject at the $F_3$, $F_4$, $F_{p1}$ and $F_{p2}$ sites, calculating SSVEP

amplitudes and phase differences from EEG signals from said electrodes, calculating values for attraction-repulsion using the equation:

$$\text{attraction} = (a_1* \text{ SSVEP phase advance at electrode } F_3 + a_2* \text{ SSVEP phase advance at electrode } F_{p1} - a_3* \text{ SSVEP phase advance at electrode } F_4 - a_4* \text{ SSVEP phase advance at electrode } F_{p2})$$

where $a_1 = a_2 = a_3 = a_4 = 1.0$
whereby said values indicate each subject's attraction or repulsion towards features of the advertisement.

**Patentansprüche**

1. Verfahren zum quantitativen Bewerten der Wirksamkeit einer audiovisuellen, visuellen und/oder akustischen Werbung, umfassend folgende Schritte:

Darbieten der Werbung mehreren Probanden gegenüber, wobei die Werbung eine Abfolge von audiovisuellen, visuellen und/oder akustischen Merkmalen aufweist, die in Abhängigkeit von der Zeit auftreten;
während des Darbietens der Werbung, Erhalten von EEG-Signalen von den Probanden von vorgegebenen Orten auf ihrer Kopfhaut;
Berechnen von SSVEP-Amplituden und -Phasendifferenzen aus EEG-Signalen, die von den vorgegebenen Orten auf der Kopfhaut erhalten wurden, um Ausgangssignale zu erhalten, die vorgegebene psychische Zustände jedes Probanden in Bezug auf die Merkmale in Abhängigkeit von der Zeit darstellen;
Anwenden eines sinusförmig variierenden visuellen Flimmerreizes auf jeden Probanden während des Darbietens der Werbung, um dadurch die Berechnung von Fourier-Koeffizienten aus den Ausgangssignalen zu ermöglichen, um dadurch die Berechnung der SSVEP-Amplituden und -Phasendifferenzen zu ermöglichen;
Kombinieren der Ausgangssignale von den Probanden, um gepoolte Ausgangssignale zu erhalten, wobei der Schritt des Kombinierens der Ausgangssignale den Schritt des Mittelns von Ausgangssignalen von jedem Probanden umfasst, wobei die SSVEP-Amplituden und -Phasendifferenzen durch folgende Gleichungen berechnet werden:

$$SSVEP_{amplitude} = \sqrt{\left( A_n^2 + B_n^2 \right)}$$

$$SSVEP_{phase} = a\tan\left( \frac{B_n}{A_n} \right)$$

wobei An und $B_n$ überlappende, geglättete Fourier-Koeffizienten sind, die mittels der folgenden Gleichung berechnet werden:

$$A_n = \sum_{i=1}^{i=N} a_{n+i} / N$$

$$B_n = \sum_{i=1}^{i=N} b_{n+i} / N$$

wobei: an und $b_n$ ein Kosinus- und ein Sinus-Fourier-Koeffizient sind, die durch folgende Gleichungen berechnet werden:

$$a_n = \frac{1}{S\Delta\tau}\sum_{i=0}^{S-1} f(nT+i\Delta\tau)\cos\left(\frac{2\pi}{T}(nT+i\Delta\tau)\right)$$

$$b_n = \frac{1}{S\Delta\tau}\sum_{i=0}^{S-1} f(nT+i\Delta\tau)\sin\left(\frac{2\pi}{T}(nT+i\Delta\tau)\right)$$

wobei:

an und $b_n$ der Kosinus- bzw. der Sinus-Fourier-Koeffizient sind, wobei:

n für den n-ten Flimmerreizzyklus steht;
S die Anzahl von Abtastungen je Flimmerreizzyklus ist;
$\Delta\tau$ das Zeitintervall zwischen Abtastungen ist;
T die Periode eines Zyklus ist; und
$f(nT+i\Delta\tau)$ das EEG-Signal (roh oder mittels ICA vorverarbeitet) ist, das von den vorgegebenen Orten auf der Kopfhaut erhalten wird; und
Anzeigen der gepoolten Ausgangssignale, um dadurch die quantitative Bewertung der Reaktionen der Probanden auf die Merkmale der Werbung zu ermöglichen, um die Wirksamkeit der Merkmale der Werbung zu bewerten;

**gekennzeichnet durch**:

Anbringen von Elektroden an der Kopfhaut jedes Probanden an den Orten $F_3$, $F_4$, $P_{p1}$ und $F_{p2}$, Berechnen von SSVEP-Amplituden und -Phasendifferenzen von den Elektroden, Berechnen von Werten für "Engagement" in Bezug auf Merkmale der Werbung durch eine gewichtete mittlere SSVEP-Phasenvorverschiebung an den Orten mittels der Gleichung:

```
„Engagement"=(b₁ * SSVEP-Phasenvorverschiebung an
Elektrode F₃ + b₂ * SSVEP-Phasenvorverschiebung an
Elektrode Pₚ₁ + b₃ * SSVEP-Phasenvorverschiebung an
Elektrode F₄ + b₄ * SSVEP-Phasenvorverschiebung an
Elektrode Fₚ₂),
```

wobei $b_1$=0, 1, $b_2$=0, 4, bs=0, 1, $b_4$=0, 4,
wobei die Werte das "Engagement" jedes Probanden in Bezug auf Merkmale der Werbung angeben; und/oder

Anbringen einer Elektrode an der Kopfhaut jedes Probanden an den Orten $F_3$, $F_4$, $F_{p1}$ und $F_{p2}$, Berechnen von SSVEP-Amplituden und -Phasendifferenzen aus EEG-Signalen von den Elektroden, Berechnen von Werten für Anziehung-Abstoßung mittels der Gleichung:

```
Anziehung = (a₁* SSVEP-Phasenvorverschiebung an
Elektrode F₃+a₂* SSVEP-Phasenvorverschiebung an
Elektrode Fₚ₁-a₃* SSVEP-Phasenvorverschiebung an
Elektrode F₄-a₄* SSVEP-Phasenvorverschiebung an
Elektrode Fₚ₂),
```

wobei $a_1$=$a_2$=$a_3$=$a_4$=1,0,
wobei die Werte die Anziehung oder Abstoßung jedes Probanden in Bezug auf Merkmale der Werbung angeben.

**2.** Verfahren nach Anspruch 1, umfassend den Schritt des gleichzeitigen Anzeigens der Werbung für die mehreren Probanden.

**3.** Verfahren nach Anspruch 2, umfassend den Schritt des Auswählens von Orten auf der Kopfhaut, um Ausgangssignale zu erhalten, welche die Bewertung von Folgendem ermöglichen:

visuelle Aufmerksamkeit für Details;
visuelle Aufmerksamkeit für globale Merkmale;
multimodale Aufmerksamkeit für Details oder Begehrtheit;
multimodale Aufmerksamkeit für globale Merkmale oder Begehrtheit;
zugehörige emotionale Intensität;
Anziehung-Abstoßung;
"Engagement"; oder
Verhaltensabsicht
in Bezug auf die Merkmale der Werbung.

**4.** Verfahren nach Anspruch 1, umfassend die folgenden Schritte:

Erhalten der SSVEP-Amplitude und -Phase von mehreren Orten auf der Kopfhaut das jedes Probanden; und
Verwenden von Umkehrabbildungsmethoden, beispielsweise von BESA, EMSA oder LORETA, um modifizierte EEG-Signale zu erzeugen, die Aktivität in tieferen Regionen des Gehirns jedes Probanden, beispielsweise dem orbitofrontalen Cortex oder dem ventromedialen Cortex, darstellen.

**5.** Verfahren nach Anspruch 1 oder 4, umfassend den Schritt des Mittelns der Fourier-Koeffizienten $A_n$ und $B_n$ für eine ausgewählte Gruppe von Probanden und dann des Berechnens der SSVEP-Amplituden und SSVEP-Phasendifferenzen für die Gruppe von Probanden.

**6.** Verfahren nach einem beliebigen der Ansprüche 1 bis 5, wobei das Flimmersignal nur auf das periphere Sehfeld jedes Probanden angewandt wird.

**7.** Verfahren nach Anspruch 6, umfassend die Schritte des Richtens des Lichts in Richtung der Augen jedes Probanden über erste und zweite Schirme, derart, dass das Licht, das durch den Schirm hindurchtritt, das Flimmersignal darstellt, und wobei jeder Schirm einen opaken Bereich umfasst, und wobei das Verfahren ferner den Schritt des Positionierens der Schirme zu der relativen Position jedes Probanden umfasst, derart, dass die opaken Bereiche verhindern, dass das Flimmersignal auf die Fovea jedes Auges jedes Probanden auftrifft.

**8.** Verfahren nach Anspruch 7, wobei die Opazität jedes Schirms in Abhängigkeit von dem Abstand von dessen opakem Bereich abnimmt, derart, dass die Intensität des Flimmersignals, das auf jede Netzhaut jedes Probanden auftrifft, dem Wert nach von dem zentralen Sehfeld zu dem peripheren Sehfeld abnimmt.

**9.** Verfahren nach Anspruch 8, umfassend den Schritt des Aufbringens eines Maskiermusters auf jeden Schirm, um dessen Opazität zu definieren, wobei das Verfahren den Schritt des Aufbringens des Musters gemäß einer Maskiermusterfunktion umfasst, die null oder niedrige Gradienten für Änderungen der Opazität an ihren opaken Bereich und periphere Bereiche davon angrenzend bereitstellt, die Teile des Flimmersignals definieren, das auf das periphere Sehfeld jedes Probanden auftrifft.

**10.** Verfahren nach Anspruch 9, wobei der opake Bereich jedes Schirms kreisförmig ist und wobei die Maskiermusterfunktion als Gauß'sche Funktion gewählt wird, derart, dass die Opazität P des Schirms durch die Gleichung:

$$P = e^{-(r-R)^2}/G^2$$

definiert wird, wobei:

r der radiale Abstand von dem Mittelpunkt des opaken Bereichs ist; und
G ein Parameter ist, der die Rate der Abnahme der Opazität mit dem radialen Abstand bestimmt, und wobei, wenn r<R, P=1.

**11.** Verfahren nach Anspruch 8, wobei G einen Wert im Bereich R/4 und 2R aufweist.

**12.** Verfahren nach Anspruch 1, umfassend die Schritte des Anbringens einer Elektrode an der Kopfhaut jedes Probanden an dem Ort $O_1$, des Berechnens von SSVEP-Amplituden und -Phasendifferenzen aus EEG-Signalen von der Elektrode, wobei die Ausgangssignale die visuelle Aufmerksamkeit jedes Probanden für Details der Werbung angeben.

**13.** Verfahren nach Anspruch 4, umfassend den Schritt des Verwendens von Umkehrabbildung bestimmt die Gehirnaktivität in dem linken Gehirncortex in der Nähe des Brodmann-Areals 17, wobei die modifizierten Ausgangssignale die visuelle Aufmerksamkeit jedes Probanden für Details der Werbung angeben.

**14.** Verfahren nach Anspruch 1, umfassend die Schritte des Anbringens einer Elektrode an der Kopfhaut jedes Probanden an dem Ort $O_2$, des Berechnens von SSVEP-Amplituden und -Phasendifferenzen aus EEG-Signalen von der Elektrode, wobei die Ausgangssignale die visuelle Aufmerksamkeit jedes Probanden für globale Merkmale der Werbung angeben.

**15.** Verfahren nach Anspruch 4, umfassend den Schritt des Verwendens von Umkehrabbildung bestimmt die Gehirnaktivität in dem rechten Gehirncortex in der Nähe des Brodmann-Areals 17, wobei die Ausgangssignale die visuelle Aufmerksamkeit jedes Probanden für globale Merkmale der Werbung angeben.

**16.** Verfahren nach Anspruch 1, umfassend den Schritt des Anbringens einer Elektrode an der Kopfhaut jedes Probanden an dem Ort $P_3$, des Berechnens von SSVEP-Amplituden und -Phasendifferenzen aus EEG-Signalen von der Elektrode, wobei die Ausgangssignale für jeden Probanden die multimodale Aufmerksamkeit für Details oder die Begehrtheit in Bezug auf Merkmale der Werbung angeben.

**17.** Verfahren nach Anspruch 4, wobei der Schritt des Verwendens von Umkehrabbildung die Gehirnaktivität in dem linken Gehirncortex in der Nähe des intraparietalen Bereichs bestimmt, wobei die Ausgangssignale für jeden Probanden die multimodale Aufmerksamkeit für Details oder die Begehrtheit in Bezug auf Merkmale der Werbung angeben.

**18.** Verfahren nach Anspruch 1, umfassend den Schritt des Anbringens einer Elektrode an der Kopfhaut jedes Probanden an dem Ort $P_4$, des Berechnens von SSVEP-Amplituden und -Phasendifferenzen aus EEG-Signalen von der Elektrode, wobei die Ausgangssignale für jeden Probanden die multimodale Aufmerksamkeit für globale Merkmale oder die Begehrtheit der Merkmale der Werbung angeben.

**19.** Verfahren nach Anspruch 4, wobei der Schritt des Verwendens von Umkehrabbildung die Gehirnaktivität in dem rechten Gehirncortex in der Nähe des intraparietalen Bereichs bestimmt, wobei die Ausgangssignale für jeden Probanden die multimodale Aufmerksamkeit für globale Merkmale oder die Begehrtheit der Merkmale der Werbung angeben.

**20.** Verfahren nach Anspruch 1, umfassend den Schritt des Anbringens einer Elektrode an der Kopfhaut jedes Probanden an einem Ort, der von den Orten $O_2$, $P_4$ und $T_6$ annähernd gleich beabstandet ist, des Berechnens von SSVEP-Amplituden und -Phasendifferenzen aus EEG-Signalen von der Elektrode, wobei die Ausgangssignale die mit der Werbung verbundene emotionale Intensität bei jedem Probanden angeben.

**21.** Verfahren nach Anspruch 4, wobei der Schritt des Verwendens von Umkehrabbildung die Gehirnaktivität in dem rechten Gehirncortex in der Nähe der rechten parietotemporalen Verbindung bestimmt, wobei die Ausgangssignale die mit der Werbung verbundene emotionale Intensität bei jedem Probanden angeben.

**22.** Verfahren nach Anspruch 4, wobei der Schritt des Verwendens von Umkehrabbildung die Gehirnaktivität bestimmt in:

dem rechten orbitofrontalen Cortex in der Nähe des Brodmann-Areals 11;
dem rechten dorsolateralen präfrontalen Cortex in der Nähe des Brodmann-Areals 9;
dem linken orbitofrontalen Cortex in der Nähe des Brodmann-Areals 11; und
dem linken dorsolateralen präfrontalen Cortex in der Nähe des Brodmann-Areals 9; und
Berechnen eines Werts für Anziehung-Abstoßung durch Verwendung der Gleichung:

Anziehung = ($c_1$*SSVEP-Phasenvorverschiebung an rechtem orbitofrontalen Cortex (in der Nähe des Brodmann-Areals 11) + $c_2$*SSVEP-Phasenvorverschiebung an rechtem dorsolateralen präfrontalen Cortex (in der Nähe des Brodmann-Areals 9) + $c_3$*SSVEP-Phasenvorverschiebung linker orbitofrontaler Cortex (in der Nähe des Brodmann-Areals 11) + $c_4$*SSVEP-Phasenvorverschiebung linker dorsolateraler präfrontaler Cortex (in der Nähe des Brodmann-Areals 9)),

wobei $c_1$=1, $c_2$=1, $c_3$=1, $c_4$=1,
wobei die Werte die Anziehung oder Abstoßung jedes Probanden in Bezug auf Merkmale der Werbung angeben.

23. Verfahren nach Anspruch 4, wobei der Schritt des Verwendens von Umkehrabbildung die Gehirnaktivität bestimmt in:

dem rechten orbitofrontalen Cortex in der Nähe des Brodmann-Areals 11;
dem rechten dorsolateralen präfrontalen Cortex in der Nähe des Brodmann-Areals 9;
dem linken frontalen Cortex in der Nähe des Brodmann-Areals 11; und
dem linken dorsolateralen präfrontalen Cortex in der Nähe des Brodmann-Areals 9;
Berechnen von SSVEP-Amplituden und -Phasendifferenzen aus den modifizierten EEG-Signalen von den Elektroden; und
Berechnen eines Werts für "Engagement" mittels der Gleichung:

„Engagement"=($d_1$*SSVEP-Phasenvorverschiebung an rechtem orbitofrontalen Cortex (in der Nähe des Brodmann-Areals 11) + $d_2$*SSVEP-Phasenvorverschiebung rechter dorsolateraler präfrontaler Cortex (in der Nähe des Brodmann-Areals 9) + $d_3$*SSVEP-Phasenvorverschiebung an linkem orbitofrontalen Cortex (in der Nähe des Brodmann-Areals 11) + $d_4$*SSVEP-Phasenvorverschiebung an linkem dorsolateralen präfrontalen Cortex (in der Nähe des Brodmann-Areals 9)),

wobei $d_1$=0, 1, $d_2$=0, 4, $d_3$=0, 1, $d_4$=0, 4,
wobei die Werte das "Engagement" jedes Probanden in Bezug auf Merkmale der Werbung angeben.

24. Verfahren nach Anspruch 1, umfassend die Schritte des Anbringens einer Elektrode an der Kopfhaut jedes Probanden an einem Ort, der von den Orten $C_3$, $F_3$ und $F_7$ annähernd gleich beabstandet ist, des Berechnens von SSVEP-Amplituden und -Phasendifferenzen aus EEG-Signalen von der Elektrode, wobei Ausgangssignale die Verhaltensabsicht jedes Probanden in Bezug auf den Gegenstand der Werbung angeben.

25. Verfahren nach Anspruch 4, wobei der Schritt des Verwendens von Umkehrabbildung die Gehirnaktivität in dem linken Gehirncortex in der Nähe der Brodmann-Areale 6, 44, 45, 46 und 47 bestimmt, Berechnen von SSVEP-Amplitude und -Phasendifferenzen aus den modifizierten EEG-Signalen von den Elektroden, wobei Ausgangssignale die Verhaltensabsicht jedes Probanden in Bezug auf den Gegenstand der Werbung angeben.

26. Verfahren nach einem beliebigen der Ansprüche 1 bis 25, wobei die gepoolten Ausgangssignale grafisch dargestellt werden.

27. Verfahren nach Anspruch 26, wobei die gepoolten Ausgangssignale auf einem Videomonitor angezeigt werden, der gleichzeitig die Werbung, die bewertet wird, anzeigt.

28. System zum quantitativen Bewerten der Wirksamkeit einer audiovisuellen, visuellen und/oder akustischen Werbung, umfassend:

Mittel zum Darbieten der Werbung mehreren Probanden gegenüber, wobei die Werbung eine Abfolge von audiovisuellen, visuellen und/oder akustischen Merkmalen aufweist, die in Abhängigkeit von der Zeit auftreten;
Mittel zum Erhalten, während des Darbietens der Werbung, von EEG-Signalen von den Probanden von vorgegebenen Orten auf ihrer Kopfhaut;
Mittel zum Berechnen von SSVEP-Amplituden und -Phasendifferenzen aus EEG-Signalen, die von den vorgegebenen Orten auf der Kopfhaut erhalten werden, um Ausgangssignale zu erhalten, die vorgegebene psychische Zustände jedes Probanden in Bezug auf Merkmale in Abhängigkeit von der Zeit darstellen;
Mittel zum Anwenden eines sinusförmig variierenden visuellen Flimmerreizes auf jeden Probanden während des Darbietens der Werbung, um dadurch die Berechnung von Fourier-Koeffizienten aus den Ausgangssignalen zu ermöglichen, um dadurch die Berechnung der SSVEP-Amplituden und -Phasendifferenzen zu ermöglichen;
Mittel zum Kombinieren der Ausgangssignale von den Probanden, um gepoolte Ausgangssignale zu erhalten, wobei der Schritt des Kombinierens der Ausgangssignale den Schritt des Mittelns von Ausgangssignalen von jedem Probanden umfasst, wobei die SSVEP-Amplituden und -Phasendifferenzen durch folgende Gleichungen berechnet werden:

$$SSVEP_{amplitude} = \sqrt{\left(A_n^2 + B_n^2\right)}$$

$$SSVEP_{phase} = a\tan\left(\frac{B_n}{A_n}\right)$$

wobei $A_n$ und $B_n$ überlappende, geglättete Fourier-Koeffizienten sind, die mittels der folgenden Gleichung berechnet werden:

$$A_n = \sum_{i=1}^{i=N} a_{n+i} / N$$

$$B_n = \sum_{i=1}^{i=N} b_{n+i} / N$$

wobei: $a_n$ und $b_n$ ein Kosinus- und ein Sinus-Fourier-Koeffizient sind, die durch folgende Gleichungen berechnet werden:

$$a_n = \frac{1}{S\Delta\tau}\sum_{i=0}^{S-1} f(nT + i\Delta\tau)\cos\left(\frac{2\pi}{T}(nT + i\Delta\tau)\right)$$

$$b_n = \frac{1}{S\Delta\tau}\sum_{i=0}^{S-1} f(nT + i\Delta\tau)\sin\left(\frac{2\pi}{T}(nT + i\Delta\tau)\right)$$

wobei:

$a_n$ und $b_n$ der Kosinus- bzw. der Sinus-Fourier-Koeffizient sind, wobei:
n für den n-ten Flimmerreizzyklus steht;
S die Anzahl von Abtastungen je Flimmerreizzyklus ist;
$\Delta\tau$ das Zeitintervall zwischen Abtastungen ist;
T die Periode eines Zyklus ist; und
$f(nT+i\Delta\tau)$ das EEG-Signal (roh oder mittels ICA vorverarbeitet) ist, das von den vorgegebenen Orten auf der Kopfhaut erhalten wird; und

Mittel zum Anzeigen der gepoolten Ausgangssignale, um dadurch die quantitative Bewertung der Reaktionen der Probanden auf die Merkmale der Werbung zu ermöglichen, um die Wirksamkeit der Merkmale der Werbung zu bewerten;

**gekennzeichnet durch**:

Mittel zum Anbringen von Elektroden an der Kopfhaut jedes Probanden an den Orten $F_3$, $F_4$, $P_{p1}$ und $F_{p2}$, Berechnen von SSVEP-Amplituden und -Phasendifferenzen von den Elektroden, Berechnen von Werten für "Engagement" in Bezug auf Merkmale der Werbung durch eine gewichtete mittlere SSVEP-Phasenvorverschiebung an den Orten mittels der Gleichung:

$$\text{„Engagement"} = (b_1 * \text{SSVEP-Phasenvorverschiebung an Elektrode } F_3 + b_2 * \text{SSVEP-Phasenvorverschiebung an Elektrode } P_{p1} + b_3 * \text{SSVEP-Phasenvorverschiebung an Elektrode } F_4 + b_4 * \text{SSVEP-Phasenvorverschiebung an Elektrode } F_{p2}),$$

wobei $b_1$=0, 1, $b_2$=0, 4, $b_3$=0, 1, $b_4$=0, 4,
wobei die Werte das "Engagement" jedes Probanden in Bezug auf Merkmale der Werbung angeben; und/oder

Mittel zum Anbringen einer Elektrode an der Kopfhaut jedes Probanden an den Orten $F_3$, $F_4$, $F_{p1}$ und $F_{p2}$, Berechnen von SSVEP-Amplituden und -Phasendifferenzen aus EEG-Signalen von den Elektroden, Berechnen von Werten für Anziehung-Abstoßung mittels der Gleichung:

$$\text{Anziehung} = (a_1 * \text{SSVEP-Phasenvorverschiebung an Elektrode } F_3 + a_2 * \text{SSVEP-Phasenvorverschiebung an Elektrode } F_{p1} - a_3 * \text{SSVEP-Phasenvorverschiebung an Elektrode } F_4 - a_4 * \text{SSVEP-Phasenvorverschiebung an Elektrode } F_{p2}),$$

wobei $a_1$=$a_2$=$a_3$=$a_4$=1, 0,
wobei die Werte die Anziehung oder Abstoßung jedes Probanden in Bezug auf Merkmale der Werbung angeben.

**Revendications**

1. Procédé d'évaluation quantitative de l'efficacité d'un message publicitaire audiovisuel, visuel et/ou audio, incluant les étapes de :

la présentation du message publicitaire à une pluralité de sujets, le message publicitaire possédant une séquence de caractéristiques audiovisuelles, visuelles et/ou audio qui apparaissent en fonction du temps ;
l'obtention, durant la présentation du message publicitaire, de signaux d'EEG des sujets à partir de sites de cuir chevelu prédéterminés de ceux-ci ;
le calcul d'amplitudes et de différences de phase de SSVEP à partir de signaux d'EEG obtenus desdits sites de cuir chevelu prédéterminés afin d'obtenir des signaux de sortie qui représentent des états psychologiques prédéterminés de chaque sujet envers lesdites caractéristiques en fonction du temps ;
l'application d'un stimulus de papillotement visuel à variation sinusoïdale sur chaque sujet durant la présentation du message publicitaire pour ainsi permettre le calcul de coefficients de Fourier à partir desdits signaux de sortie pour ainsi permettre le calcul desdites amplitudes et différences de phase de SSVEP ;
la combinaison des signaux de sortie desdits sujets pour obtenir des signaux de sortie collectifs, dans lequel l'étape de la combinaison des signaux de sortie inclut l'étape de la réalisation de moyenne de signaux de sortie de chaque sujet, dans lequel lesdites amplitudes et différences de phase de SSVEP sont calculées par les équations :

$$SSVEP_{amplitude} = \sqrt{(A_n^2 + B_n^2)}$$

$$SSVEP_{phase} = \operatorname{a} \tan\left(\frac{B_n}{A_n}\right)$$

dans lequel $A_n$ et $B_n$ sont des coefficients de Fourier lissés se chevauchant calculés en utilisant l'équation :

$$A_n = \sum_{i=1}^{i=N} a_{n+i} \Big/ N$$

$$B_n = \sum_{i=1}^{i=N} b_{n+i} \Big/ N$$

où : $a_n$ et $b_n$ sont des coefficients de Fourier cosinusoïdal et sinusoïdal calculés par les équations :

$$a_n = \frac{1}{S\Delta\tau} \sum_{i=0}^{S-1} f(nT + i\Delta\tau) \cos\left(\frac{2\pi}{T}(nT + i\Delta\tau)\right)$$

$$b_n = \frac{1}{S\Delta\tau} \sum_{i=0}^{S-1} f(nT + i\Delta\tau) \sin\left(\frac{2\pi}{T}(nT + i\Delta\tau)\right)$$

où :

$a_n$ et $b_n$ sont les coefficients de Fourier cosinusoïdal et sinusoïdal respectivement où :
n représente le n-ième cycle de stimulus de papillotement ;
S est le nombre d'échantillons par cycle de stimulus de papillotement ;
$\Delta\tau$ est l'intervalle de temps entre des échantillons ;
T est la période d'un cycle ; et
f(nT+i$\Delta\tau$) est le signal d'EEG (brut ou prétraité en utilisant ICA) obtenu desdits sites de cuir chevelu prédéterminés ; et
l'affichage des signaux de sortie collectifs pour ainsi permettre l'évaluation quantitative des réponses des sujets auxdites caractéristiques du message publicitaire afin d'évaluer l'efficacité des caractéristiques du message publicitaire ;

**caractérisé par** :

l'application d'électrodes sur le cuir chevelu de chaque sujet à des sites $F_3$, $F_4$, $P_{p1}$ et $F_{P2}$, le calcul d'amplitudes et de différences de phase de SSVEP desdites électrodes, le calcul de valeurs pour l'engagement envers des caractéristiques du message publicitaire par une avance de phase de SSVEP moyenne pondérée auxdits sites en utilisant l'équation :

```
engagement = (b₁ * avance  de  phase  de  SSVEP  à
l'électrode  F₃ + b₂ * avance  de  phase  de  SSVEP  à
l'électrode  Pₚ₁ + b₃ * avance  de  phase  de  SSVEP  à
l'électrode  F₄ + b₄ * avance  de  phase  de  SSVEP  à
l'électrode Fₚ₂)
```

où $b_1 = 0,1$, $b_2 = 0,4$, $b_3 = 0,1$, $b_4 = 0,4$,
moyennant quoi lesdites valeurs indiquent l'engagement de chaque sujet envers des caractéristiques du message publicitaire ; et/ou

l'application d'une électrode sur le cuir chevelu de chaque sujet aux sites $F_3$, $F_4$, $F_{p1}$ et $F_{P2}$, le calcul d'amplitudes et de différences de phase de SSVEP à partir de signaux d'EEG desdites électrodes, le calcul de valeurs pour l'attraction-la répulsion en utilisant l'équation :

```
attraction = (a₁ * avance  de  phase  de  SSVEP  à
l'électrode  F₃ + a₂ * avance  de  phase  de  SSVEP  à
l'électrode  Fₚ₁ − a₃ * avance  de  phase  de  SSVEP  à
l'électrode  F₄ − a₄ * avance  de  phase  de  SSVEP  à
l'électrode Fₚ₂)
```

où $a_1 = a_2 = a_3 = a_4 = 1,0$
moyennant quoi lesdites valeurs indiquent l'attraction ou la répulsion de chaque sujet envers des caractéristiques du message publicitaire.

**2.** Procédé selon la revendication 1, incluant l'étape de la présentation simultanée du message publicitaire à ladite pluralité de sujets.

**3.** Procédé selon la revendication 2, incluant l'étape de la sélection de sites de cuir chevelu afin d'obtenir des signaux de sortie qui permettent l'évaluation de :

l'attention visuelle au détail ;
l'attention visuelle à des caractéristiques globales ;
l'attention multimodale au détail ou la désirabilité ;
l'attention multimodale à des caractéristiques globales ou la désirabilité ;
l'intensité émotionnelle associée ;
l'attraction-la répulsion ;
l'engagement ; ou
l'intention comportementale
par rapport aux caractéristiques du message publicitaire.

**4.** Procédé selon la revendication 1, incluant les étapes de :

l'obtention d'amplitude et de phase de SSVEP à partir d'une pluralité de sites de cuir chevelu de chaque sujet ; et l'utilisation de techniques de mappage inverse telles que BESA, EMSA ou LORETA pour produire des signaux d'EEG modifiés qui représentent l'activité dans des régions plus profondes du cerveau de chaque sujet, telles que le cortex orbito-frontal ou le cortex ventro-médial.

5. Procédé selon la revendication 1 ou 4, incluant l'étape de la réalisation de moyenne des coefficients de Fourier $A_n$ et $B_n$ pour un groupe sélectionné de sujets et puis le calcul des amplitudes de SSVEP et des différences de phase de SSVEP pour ledit groupe de sujets.

6. Procédé selon l'une quelconque des revendications 1 à 5, dans lequel le signal de papillotement est appliqué seulement sur la vision périphérique de chaque sujet.

7. Procédé selon la revendication 6, incluant les étapes de la direction de la lumière vers les yeux de chaque sujet par l'intermédiaire de premier et second écrans pour que la lumière passant à travers l'écran constitue ledit signal de papillotement et dans lequel chaque écran inclut une zone opaque, et dans lequel le procédé inclut en outre l'étape du positionnement des écrans par rapport à la position relative de chaque sujet de telle sorte que lesdites zones opaques empêchent ledit signal de papillotement d'être incident sur la fovéa de chaque oeil de chaque sujet.

8. Procédé selon la revendication 7, dans lequel l'opacité de chaque écran diminue en fonction de la distance à partir de sa zone opaque pour que la valeur de l'intensité du signal de papillotement incident sur chaque rétine de chaque sujet diminue de la vision centrale à la vision périphérique.

9. Procédé selon la revendication 8, incluant l'étape de l'application d'un motif de masquage sur chaque écran pour définir l'opacité de celui-ci, le procédé incluant l'étape de l'application du motif conformément à une fonction de motif de masquage qui fournit des gradients nuls ou bas pour des changements d'opacité adjacents à sa zone opaque et des zones périphériques de celui-ci qui définissent des parties du signal de papillotement incident sur la vision périphérique de chaque sujet.

10. Procédé selon la revendication 9, dans lequel la zone opaque de chaque écran est circulaire et dans lequel la fonction de motif de masquage est sélectionnée pour être une fonction gaussienne, pour que l'opacité P de l'écran soit définie par l'équation :

$$P = e^{-(r-R)^2/G^2}$$

où :

r est la distance radiale à partir du centre de la zone opaque ; et
G est un paramètre qui détermine le taux d'affaiblissement d'opacité avec la distance radiale, et dans lequel lorsque r < R, P = 1.

11. Procédé selon la revendication 8 dans lequel G possède une valeur dans la plage R/4 et 2R.

12. Procédé selon la revendication 1, incluant les étapes de l'application d'une électrode sur le cuir chevelu de chaque sujet au site $O_1$, le calcul d'amplitudes et de différences de phase de SSVEP à partir de signaux d'EEG de ladite électrode moyennant quoi les signaux de sortie indiquent l'attention visuelle de chaque sujet à des détails du message publicitaire.

13. Procédé selon la revendication 4, incluant l'étape de l'utilisation de mappage inverse détermine l'activité cérébrale dans le cortex cérébral gauche dans le voisinage de l'aire de Brodmann 17 moyennant quoi les signaux de sortie modifiés indiquent l'attention visuelle de chaque sujet à des détails du message publicitaire.

14. Procédé selon la revendication 1, incluant les étapes de l'application d'une électrode sur le cuir chevelu de chaque sujet au site $O_2$, le calcul d'amplitudes et de différences de phase de SSVEP à partir de signaux d'EEG de ladite électrode moyennant quoi les signaux de sortie sont indicatifs de l'attention visuelle de chaque sujet à des caractéristiques globales du message publicitaire.

15. Procédé selon la revendication 4, incluant l'étape de l'utilisation de mappage inverse détermine activité cérébrale dans le cortex cérébral droit dans le voisinage de l'aire de Brodmann 17 moyennant quoi les signaux de sortie indiquent l'attention visuelle de chaque sujet à des caractéristiques globales du message publicitaire.

**16.** Procédé selon la revendication 1, incluant l'étape de l'application d'une électrode sur le cuir chevelu de chaque sujet au site $P_3$, le calcul d'amplitudes et de différences de phase de SSVEP à partir de signaux d'EEG de ladite électrode moyennant quoi les signaux de sortie sont indicatifs de l'attention multimodale de chaque sujet au détail ou de la désirabilité envers des caractéristiques du message publicitaire.

**17.** Procédé selon la revendication 4, dans lequel l'étape de l'utilisation de mappage inverse détermine l'activité cérébrale dans le cortex cérébral gauche dans le voisinage de l'aire intrapariétale moyennant quoi les signaux de sortie indiquent l'attention multimodale de chaque sujet au détail ou la désirabilité des caractéristiques du message publicitaire.

**18.** Procédé selon la revendication 1, incluant l'étape de l'application d'une électrode sur le cuir chevelu de chaque sujet au site $P_4$, le calcul d'amplitudes et de différences de phase de SSVEP à partir de signaux d'EEG de ladite électrode moyennant quoi les signaux de sortie indiquent l'attention multimodale de chaque sujet à des caractéristiques globales ou la désirabilité des caractéristiques du message publicitaire.

**19.** Procédé selon la revendication 4, dans lequel l'étape de l'utilisation de mappage inverse détermine l'activité cérébrale dans le cortex cérébral droit dans le voisinage de l'aire intrapariétale moyennant quoi les signaux de sortie indiquent l'attention multimodale de chaque sujet à des caractéristiques globales ou la désirabilité des caractéristiques du message publicitaire.

**20.** Procédé selon la revendication 1, incluant l'étape de l'application d'une électrode sur le cuir chevelu de chaque sujet à un site qui est approximativement équidistant des sites $O_2$, $P_4$ et $T_6$, le calcul d'amplitudes et de différences de phase de SSVEP à partir de signaux d'EEG de ladite électrode moyennant quoi les signaux de sortie indiquent l'intensité émotionnelle de chaque sujet associée au message publicitaire.

**21.** Procédé selon la revendication 4, dans lequel l'étape de l'utilisation de mappage inverse détermine l'activité cérébrale dans le cortex cérébral droit dans le voisinage de la jonction pariéto-temporale droite moyennant quoi les signaux de sortie indiquent l'intensité émotionnelle de chaque sujet associée au message publicitaire.

**22.** Procédé selon la revendication 4 dans lequel l'étape de l'utilisation de mappage inverse détermine l'activité cérébrale dans :

le cortex orbito-frontal droit dans le voisinage de l'aire de Brodmann 11 ;
le cortex préfrontal dorso-latéral droit dans le voisinage de l'aire de Brodmann 9 ;
le cortex orbito-frontal gauche dans le voisinage de l'aire de Brodmann 11 ; et
le cortex préfrontal dorso-latéral gauche dans le voisinage de l'aire de Brodmann 9 ; et
le calcul d'une valeur pour l'attraction-la répulsion en utilisant l'équation :

```
attraction = (c₁ * avance de phase de SSVEP dans le
cortex orbito-frontal droit (dans le voisinage de l'aire
de Brodmann 11) + c₂ * avance de phase de SSVEP dans le
cortex préfrontal dorso-latéral droit (dans le voisinage
de l'aire de Brodmann 9) + c₃ * avance de phase de SSVEP
le cortex orbito-frontal gauche (dans le voisinage de
l'aire de Brodmann 11) + c₄ * avance de phase de SSVEP le
cortex préfrontal dorso-latéral gauche (voisinage de
l'aire de Brodmann 9))
```

où $C_1 = 1$, $C_2 = 1$, $C_3 = 1$, $C_4 = 1$,
moyennant quoi lesdites valeurs indiquent l'attraction ou la répulsion de chaque sujet envers des caractéristiques du message publicitaire.

**23.** Procédé selon la revendication 4, dans lequel l'étape de l'utilisation de mappage inverse détermine l'activité cérébrale dans :

le cortex orbito-frontal droit dans le voisinage de l'aire de Brodmann 11 ;
le cortex préfrontal dorso-latéral droit dans le voisinage de l'aire de Brodmann 9 ;
le cortex frontal gauche dans le voisinage de l'aire de Brodmann 11 ; et
le cortex préfrontal dorso-latéral gauche dans le voisinage de l'aire de Brodmann 9,
le calcul d'amplitudes et de différences de phase de SSVEP à partir des signaux d'EEG modifiés desdites électrodes ; et
le calcul d'une valeur pour l'engagement en utilisant l'équation :

```
engagement = (d₁ * avance de phase de SSVEP dans le
cortex orbito-frontal droit (dans le voisinage de l'aire
de Brodmann 11) + d₂ * avance de phase de SSVEP le cortex
préfrontal dorso-latéral droit (dans le voisinage de
l'aire de Brodmann 9) + d₃ * avance de phase de SSVEP
dans le cortex orbito-frontal gauche (dans le voisinage
de l'aire de Brodmann 11) + d₄ * avance de phase de SSVEP
dans le cortex préfrontal dorso-latéral gauche (dans le
voisinage de l'aire de Brodmann 9))
```

où $d_1 = 0,1$, $d_2 = 0,4$, $d_3 = 0,1$, $d_4 = 0,4$,
moyennant quoi lesdites valeurs indiquent l'engagement de chaque sujet envers des caractéristiques du message publicitaire.

24. Procédé selon la revendication 1 incluant les étapes de l'application d'une électrode sur le cuir chevelu de chaque sujet à un site approximativement équidistant des sites $C_3$, $F_3$ et $F_7$, le calcul d'amplitudes et de différences de phase de SSVEP à partir de signaux d'EEG de ladite électrode, moyennant quoi des signaux de sortie indiquent l'intention comportementale de chaque sujet envers le sujet du message publicitaire.

25. Procédé selon la revendication 4, dans lequel l'étape de l'utilisation de mappage inverse détermine l'activité cérébrale dans le cortex cérébral gauche dans le voisinage des aires de Brodmann 6, 44, 45, 46 et 47, le calcul d'amplitudes et de différences de phase de SSVEP à partir desdits signaux d'EEG modifiés desdites électrodes moyennant quoi des signaux de sortie indiquent l'intention comportementale de chaque sujet envers le sujet du message publicitaire.

26. Procédé selon l'une quelconque des revendications 1 à 25, dans lequel lesdits signaux de sortie collectifs sont affichés graphiquement.

27. Procédé selon la revendication 26, dans lequel lesdits signaux de sortie collectifs sont affichés sur un moniteur vidéo qui affiche simultanément le message publicitaire en train d'être évalué.

28. Système pour quantitativement évaluer l'efficacité d'un message publicitaire audiovisuel, visuel et/ou audio, incluant :

des moyens pour la présentation du message publicitaire à une pluralité de sujets, le message publicitaire possédant une séquence de caractéristiques audiovisuelles, visuelles et/ou audio qui apparaissent en fonction du temps ;
des moyens pour l'obtention, durant la présentation du message publicitaire, de signaux d'EEG des sujets à partir de sites de cuir chevelu prédéterminés de ceux-ci ;
des moyens pour le calcul d'amplitudes et de différences de phase de SSVEP à partir de signaux d'EEG obtenus desdits sites de cuir chevelu prédéterminés afin d'obtenir des signaux de sortie qui représentent des états psychologiques prédéterminés de chaque sujet envers lesdites caractéristiques en fonction du temps ;
des moyens pour l'application d'un stimulus de papillotement visuel à variation sinusoïdale sur chaque sujet durant la présentation du message publicitaire pour ainsi permettre le calcul de coefficients de Fourier à partir desdits signaux de sortie pour ainsi permettre le calcul desdites amplitudes et différences de phase de SSVEP ;

des moyens pour la combinaison des signaux de sortie desdits sujets pour obtenir des signaux de sortie collectifs, dans lequel l'étape de la combinaison des signaux de sortie inclut l'étape de la réalisation de moyenne de signaux de sortie de chaque sujet, dans lequel lesdites amplitudes et différences de phase de SSVEP sont calculées par les équations :

$$SSVEP_{amplitude} = \sqrt{(A_n^2 + B_n^2)}$$

$$SSVEP_{phase} = a\tan\left(\frac{B_n}{A_n}\right)$$

dans lequel $A_n$ et $B_n$ sont des coefficients de Fourier lissés se chevauchant calculés en utilisant l'équation :

$$A_n = \sum_{i=1}^{i=N} a_{n+i}\Big/ N$$

$$B_n = \sum_{i=1}^{i=N} b_{n+i}\Big/ N$$

où : $a_n$ et $b_n$ sont des coefficients de Fourier cosinusoïdal et sinusoïdal calculés par les équations :

$$a_n = \frac{1}{S\Delta\tau}\sum_{i=0}^{S-1} f(nT + i\Delta\tau)\cos\left(\frac{2\pi}{T}(nT + i\Delta\tau)\right)$$

$$b_n = \frac{1}{S\Delta\tau}\sum_{i=0}^{S-1} f(nT + i\Delta\tau)\sin\left(\frac{2\pi}{T}(nT + i\Delta\tau)\right)$$

où :

$a_n$ et $b_n$ sont les coefficients de Fourier cosinusoïdal et sinusoïdal respectivement où :

n représente le n-ième cycle de stimulus de papillotement ;
S est le nombre d'échantillons par cycle de stimulus de papillotement ;
$\Delta\tau$ est l'intervalle de temps entre échantillons ;
T est la période d'un cycle ; et
$f(nT+i\Delta\tau)$ est le signal d'EEG (brut ou prétraité en utilisant ICA) obtenu desdits sites de cuir chevelu prédéterminés ; et

des moyens pour l'affichage des signaux de sortie collectifs pour ainsi permettre l'évaluation quantitative des réponses des sujets auxdites caractéristiques du message publicitaire afin d'évaluer l'efficacité des caractéristiques du message publicitaire ;

**caractérisé par** :

des moyens pour l'application d'électrodes sur le cuir chevelu de chaque sujet à des sites $F_3$, $F_4$, $P_{p1}$ et $F_{p2}$, le calcul d'amplitudes et de différences de phase de SSVEP desdites électrodes, le calcul de valeurs pour l'engagement envers des caractéristiques du message publicitaire par une avance de phase de SSVEP moyenne pondérée auxdits sites en utilisant l'équation :

```
        engagement = (b₁ * avance  de  phase  de  SSVEP  à
l'électrode  F₃ + b₂ * avance  de  phase  de  SSVEP  à
l'électrode  P_p1 + b₃ * avance  de  phase  de  SSVEP  à
l'électrode  F₄ + b₄ * avance  de  phase  de  SSVEP  à
l'électrode F_p2)
```

où $b_1 = 0, 1$, $b_2 = 0,4$, $b_3 = 0, 1$, $b_4 = 0, 4$,
moyennant quoi lesdites valeurs indiquent l'engagement de chaque sujet envers des caractéristiques du message publicitaire ; et/ou

des moyens pour l'application d'une électrode sur le cuir chevelu de chaque sujet aux sites $F_3$, $F_4$, $F_{p1}$ et $F_{p2}$, le calcul d'amplitudes et de différences de phase de SSVEP à partir de signaux d'EEG desdites électrodes, le calcul de valeurs pour l'attraction-la répulsion en utilisant l'équation :

```
        attraction = (a₁ *  avance  de  phase  de  SSVEP  à
l'électrode  F₃ + a₂ *  avance  de  phase  de  SSVEP  à
l'électrode  F_p1 − a₃ *  avance  de  phase  de  SSVEP  à
l'électrode  F₄ − a₄ *  avance  de  phase  de  SSVEP  à
l'électrode F_p2)
```

où $a_1 = a_2 = a_3 = a_4 = 1, 0$
moyennant quoi lesdites valeurs indiquent l'attraction ou la répulsion de chaque sujet envers des caracté-ristiques du message publicitaire.

Fig 1.

FIG. 2

International 10-20 system of electrode locations.

FIG. 3

FIG. 4

FIG. 5

Fig 6

Fig 7

Fig 8

Fig 9

**Long Term Memory**

*78*
*58*
*56*
*72*

**Multi-modal attention**

Fig 10

*68*
*60*
*62*

**Visual attention**

Fig 11

*80*
*64*
*66*

## REFERENCES CITED IN THE DESCRIPTION

### Patent documents cited in the description

- US 4955938 A
- US 5331969 A
- US 6792304 B
- WO 9959470 A1
- US 2006184058 A1

### Non-patent literature cited in the description

- **JOHN R. ROSSITER ; RICHARD B. SILBERSTEIN.** Brain-Imaging Detection of Visual Scene Encoding in Long-Term Memory for TV Commercials. *Journal of Advertising Research,* March 2001, 13-21
- **RICHARD B. SILBERSTEIN ; PHILIP G. HARRIS ; GEOFFREY A. NIELD ; ANDREW PIPINGAS.** Frontal Steady-State Potential Changes Predict Long-Term Recognition Memory Performance. *International Journal of Psycho Physiology,* 2000, vol. 39, 79-85
- **BELL A.J. ; SEJNOWSKI T.J.** An information maximisation approach to blind separation and blind deconvolution. *Neural Computation,* 1995, vol. 7 (6), 1129-1159
- **T-P. JUNG ; S. MAKEIG ; M. WESTERFIELD ; J. TOWHSEHD ; E. COURCHESHE ; T.J. SEJNOWSKIK.** Independent components analysis of single-trial event-related potential. *Human Brain Mapping,* 2001, vol. 14 (3), 168-85
- **JASPER HH.** *Electroenceph. Clin. Neurophysiol.,* 1958, vol. 10, 370-1